(19) **Europäisches Patentamt / European Patent Office / Office européen des brevets**

(11) **EP 4 772 535 A1**

(12) **EUROPEAN PATENT APPLICATION**
published in accordance with Art. 153(4) EPC

(43) Date of publication:
08.07.2026 Bulletin 2026/28

(21) Application number: 24858473.2

(22) Date of filing: 23.08.2024

(51) International Patent Classification (IPC):
$C07K\ 16/28^{(2006.01)}$   $C12N\ 15/13^{(2006.01)}$
$A61K\ 39/395^{(2006.01)}$   $A61P\ 35/00^{(2006.01)}$
$A61P\ 35/02^{(2006.01)}$

(52) Cooperative Patent Classification (CPC):
A61K 39/395; A61P 35/00; A61P 35/02;
C07K 16/00; C07K 16/28

(86) International application number:
PCT/CN2024/114289

(87) International publication number:
WO 2025/044937 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.08.2023 CN 202311120423

(71) Applicant: Chia Tai Tianqing Pharmaceutical
Group Co., Ltd.
Lianyungang, Jiangsu 222062 (CN)

(72) Inventors:
• MA, Yimin
Lianyungang, Jiangsu 222062 (CN)
• ZHEN, Zipeng
Lianyungang, Jiangsu 222062 (CN)
• YU, Nianqin
Lianyungang, Jiangsu 222062 (CN)
• ZHOU, Yaping
Lianyungang, Jiangsu 222062 (CN)
• CHEN, Hongmei
Lianyungang, Jiangsu 222062 (CN)
• YAO, Jiangning
Lianyungang, Jiangsu 222062 (CN)

(74) Representative: Mewburn Ellis LLP
Aurora Building
Counterslip
Bristol BS1 6BX (GB)

Remarks:
The complete document including Reference
Table(s) and the Sequence Listing(s) can be
downloaded from the EPO website

(54) **ANTIBODY TARGETING RECEPTOR TYROSINE KINASE-LIKE ORPHAN RECEPTOR 1 AND USE THEREOF**

(57) Provided are an antibody targeting receptor tyrosine kinase-like orphan receptor 1 and a use thereof. Further provided is a pharmaceutical composition comprising the antibody and a use thereof. The provided antibody achieves excellent anti-tumor activity and/or good safety. The provided antibody can be used for the treatment of tumors.

FIG. 1B

**Description**

**TECHNICAL FIELD**

The present disclosure pertains to the technical field of biology, particularly relates to an antibody targeting receptor tyrosine kinase-like orphan receptor 1 or an antigen-binding fragment thereof, and further relates to use of the antibody or the antigen-binding fragment thereof.

**BACKGROUND**

Receptor tyrosine kinase-like orphan receptor 1 (ROR1) is a member of the ROR receptor family, which belongs to the cell tight junction protein family. This family consists of two closely related type I transmembrane proteins, ROR1 and ROR2, which are involved in processes such as intercellular signal communication and intracellular signal transduction, regulating cell proliferation, differentiation, and migration. The structure of ROR1 includes an extracellular region, a transmembrane region, and an intracellular region. The extracellular domain consists of an Ig-like domain, a cysteine-rich frizzled domain, and a kringle domain, while the intracellular domain consists of a tyrosine kinase domain, a serine/-threonine-rich region, and a proline-rich region.

ROR1 is selectively overexpressed in many solid and hematologic malignancies, such as leukemia, breast cancer, ovarian cancer, and lung adenocarcinoma, but is not significantly expressed in normal adult tissues. Multiple studies have demonstrated that ROR1 can promote tumor growth both *in vivo* and *in vitro,* playing a facilitating role in tumor malignant progression. This has established ROR1 as a potential target for cancer-targeted therapy.

Some antibodies targeting ROR1 are currently under development, but they remain limited. There is still a clinical need for more antibodies against ROR1.

**BRIEF SUMMARY**

The present disclosure provides an anti-ROR1 antibody and an antigen-binding fragment thereof, and also provides a related nucleic acid encoding the provided antibody or antigen-binding fragment thereof, a vector, a cell, a pharmaceutical composition, a preparation method, and use.

In one aspect, the present disclosure provides an anti-ROR1 antibody or an antigen-binding fragment thereof, which comprises:

(1) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(2) an HCDR1 comprising the amino acid sequence set forth in SEQ ID NO: 9, an HCDR2 comprising the amino acid sequence set forth in SEQ ID NO: 50, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(3) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(4) an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(5) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22; or

(6) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

**EP 4 772 535 A1**

In one aspect, the present disclosure provides an anti-ROR1 antibody or an antigen-binding fragment thereof, which comprises:

(1) an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8;

(2) an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16;

(3) an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24;

(4) an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32;

(5) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 53;

(6) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 54;

(7) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 55;

(8) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 53;

(9) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 54;

(10) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 55;

(11) an HCDR1, an HCDR2, and an HCDR3 in a heavy chain variable region having the amino acid sequence set forth in SEQ ID NO: 56, and an LCDR1, an LCDR2, and an LCDR3 in a light chain variable region having the amino acid sequence set forth in SEQ ID NO: 59;

(12) an HCDR1, an HCDR2, and an HCDR3 in a heavy chain variable region having the amino acid sequence set forth in SEQ ID NO: 57, and an LCDR1, an LCDR2, and an LCDR3 in a light chain variable region having the amino acid sequence set forth in SEQ ID NO: 59;

(13) an HCDR1, an HCDR2, and an HCDR3 in a heavy chain variable region having the amino acid sequence set forth in SEQ ID NO: 58, and an LCDR1, an LCDR2, and an LCDR3 in a light chain variable region having the amino acid sequence set forth in SEQ ID NO: 59;

(14) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 62;

(15) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 63;

(16) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 64;

(17) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 62;

(18) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 63; or

(19) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 64.

In one aspect, the present disclosure provides an isolated nucleic acid, which comprises a nucleotide sequence encoding the anti-ROR1 antibody or the antigen-binding fragment thereof described in the present disclosure.

In one aspect, the present disclosure provides a vector, which comprises the nucleic acid described in the present disclosure.

In one aspect, the present disclosure provides a host cell, which comprises the nucleic acid or the vector described in the present disclosure.

In one aspect, the present disclosure provides an antibody-drug conjugate, which comprises the anti-ROR1 antibody or the antigen-binding fragment thereof of the present disclosure.

In one aspect, the present disclosure provides an anti-ROR1 chimeric antigen receptor, which comprises the anti-ROR1 antibody or the antigen-binding fragment thereof of the present disclosure.

In one aspect, the present disclosure provides an engineered immune effector cell, which comprises the chimeric antigen receptor of the present disclosure.

In another aspect, the present disclosure provides a method for preparing the anti-ROR1 antibody or the antigen-binding fragment thereof described in the present disclosure, which comprises culturing the host cell to express the ROR1 antibody or the antigen-binding fragment thereof, and isolating and purifying the anti-ROR1 antibody or the antigen-binding fragment thereof in the system.

In another aspect, the present disclosure provides a pharmaceutical composition, which comprises the anti-ROR1 antibody or the antigen-binding fragment thereof, the fusion protein, the antibody-drug conjugate, or the engineered immune effector cell, and a pharmaceutically acceptable excipient.

In another aspect, the present disclosure provides use of the anti-ROR1 antibody or the antigen-binding fragment thereof, the fusion protein, the antibody-drug conjugate, the engineered immune effector cell, or the pharmaceutical composition for preparing a medicament for treating a disease expressing ROR1.

In another aspect, the present disclosure provides a method for treating a disease expressing ROR1, which comprises administering to a subject in need the anti-ROR1 antibody or the antigen-binding fragment thereof, the antibody-drug conjugate, the engineered immune effector cell, or the pharmaceutical composition. In another aspect, the present disclosure provides a method for treating a disease expressing ROR1, which comprises administering to a subject in need a therapeutically effective amount of the anti-ROR1 antibody or the antigen-binding fragment thereof, the antibody-drug conjugate, the engineered immune effector cell, or the pharmaceutical composition.

The anti-ROR1 antibody or the antigen-binding fragment thereof provided in the present disclosure exhibits good anti-tumor effects and/or safety.

## BRIEF DESCRIPTION OF THE DRAWINGS

FIGs. 1A-1C show the binding curves of chimeric anti-ROR1 antibodies to cells expressing ROR1 assayed by FACS, wherein FIG. 1A is for A549 cells, FIG. 1B is for Jeko-1 cells, and FIG. 1C is for SW620 cells;

FIGs. 2A-2I show the binding curves of humanized anti-ROR1 antibodies to cells expressing ROR1 assayed by FACS, wherein FIGs. 2A, 2D, and 2G are for PA-1 cells, FIGs. 2B, 2E, and 2H are for Jeko-1 cells, and FIGs. 2C, 2F, and 2I are for SW620 cells;

FIGs. 3A-3C show the internalization effects of humanized anti-ROR1 antibodies in cells expressing ROR1 assayed by FACS, wherein FIG. 3A is for NCI-H1993-ROR1 cells, FIG. 3B is for MDA-MB-468-ROR1 cells, and FIG. 3C is for NCI-H1975-ROR1 cells;

FIG. 4 shows the inhibition rates of humanized anti-ROR1 antibodies against the Wnt5a-stimulated non-classical Wnt signaling pathway in 293T-ROR1-NF-$\kappa$B cells;

FIG. 5 shows the ADCC effects of humanized anti-ROR1 antibodies on PA-1 cells.

## SUMMARY

### *Definitions and Description*

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "antibody" is used in its broadest sense and encompasses natural antibodies and artificial antibodies of various structures, including, but not limited to, various antibody structures such as monoclonal antibodies, polyclonal antibodies, monospecific antibodies, multispecific antibodies (e.g., bispecific antibodies or trispecific antibodies), and single-chain antibodies, so long as they exhibit the desired antigen-binding activity.

The term "antigen-binding fragment" refers to one or more fragments of an antibody that retain the functionality of specifically binding to an antigen. Examples encompassed within the term "antigen-binding fragment" include: (i) Fab fragments: monovalent fragments consisting of the $V_L$, $V_H$, CL, and CH1 domains; (ii) F(ab')2 fragments, bivalent fragments comprising two Fab fragments linked by a disulfide bridge in the hinge region; (iii) Fd fragments consisting of the VH and CH1 domains; (iv) Fv fragments consisting of the VL and VH domains of a single arm of the antibody; (v) dAb fragments consisting of the VH domain (see Ward et al., Nature. 341:544-546 (1989)); (vi) isolated complementarity determining regions (CDRs); (vii) nanobodies; and single-chain Fv (scFv).

The term "variable domain" or "variable region" refers to a domain of an antibody that is involved in the binding of the antibody to an antigen. For example, a natural four-chain antibody (e.g., one derived from a human, a murine, or other mammals) comprises a heavy chain variable region (also referred to as heavy chain variable domain, VH, or VH domain) and a light chain variable region (also referred to as light chain variable domain, VL, or VL domain). In most cases, each variable domain of a natural antibody consists substantially of four "framework regions (FRs)" and three "complementarity determining regions (CDRs)". The four framework regions are referred to as framework region 1 (or FR1), framework region 2 (or FR2), framework region 3 (or FR3), and framework region 4 (or FR4). The framework regions are separated by three complementarity determining regions referred to in the art and hereinafter as complementarity determining region 1 (or CDR1), complementarity determining region 2 (or CDR2), and complementarity determining region 3 (or CDR3). Thus, the general structure of a variable domain can be represented as follows: FR1-CDR1-FR2-CDR2-FR3-CDR3-FR4. Variable domains impart specificity for an antigen to an antibody by virtue of having an antigen-binding site.

The term "complementarity determining region" (CDR) is also referred to as "hypervariable region" (HVR). A natural four-chain antibody typically comprises six CDRs, among which three are in the heavy chain variable region, i.e., heavy chain CDR1 (HCDR1), heavy chain CDR2 (HCDR2), and heavy chain CDR3 (HCDR3), and three are in the light chain variable region, i.e., light chain CDR1 (LCDR1), light chain CDR2 (LCDR2), and light chain CDR3 (LCDR3).

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. The "Contact" defines CDRs based on the analysis of the crystal structure of available complexes. Additionally, alternative definitions such as IMGT and CCG are also recognized. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined by specific CDR sequences, the scope of the antibody also encompasses antibodies defined by CDR sequences under any other definitions (e.g., one of or a combination of several of the definitions such as Kabat, IMGT, Chothia, Contact, AbM, CCG).

The term "treating" or "treatment" means administering the compound (e.g., an antibody) or pharmaceutical composition described in the present disclosure to prevent, ameliorate, or eliminate a disease or one or more symptoms associated with the disease, including but not limited to: (i) preventing the occurrence of a disease or disease state in a mammal, particularly when such a mammal is predisposed to the disease state but has not yet been diagnosed with it; (ii) inhibiting a disease or disease state, i.e., arresting its progression; (iii) alleviating a disease or disease state, i.e., causing its regression; (iv) reducing any direct or indirect pathological consequences of a disease or disease state.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the anti-ROR1 antibody or the antigen-binding fragment thereof, the antibody-drug conjugate, the engineered immune effector cell, the fusion protein, or the pharmaceutical composition of the present disclosure that constitutes a "therapeutically effective amount" may vary depending on factors such as the compound or the pharmaceutical composition and its ability to elicit a desired response in an individual, the disease state and its severity, the mode of administration, and the age, sex, and weight of the mammal to be treated. The therapeutically effective amount may also be determined routinely by those skilled in the art following their knowledge and the content of the present disclosure.

The term "pharmaceutically acceptable" is used herein for those compounds, materials, compositions, and/or dosage forms, etc., that are, within the scope of sound medical judgment, suitable for use in contact with the tissues of humans and animals without excessive toxicity, irritation, allergic response, or other problems or complications, and commensurate with a reasonable benefit/risk ratio.

The term "excipient" refers to any ingredient other than the active ingredient (e.g., the antibody of the present disclosure). The selection of the excipient will largely depend on factors such as the particular mode of administration, the effect of the excipient on solubility and stability, and the nature of the dosage form.

The term "isolated" means that a target compound, such as an antibody or an antigen-binding fragment thereof, or a nucleic acid, has been isolated from its natural environment.

The terms "$X_n$" and "Xaa" are equivalent and refer to an unspecified amino acid whose scope is specified by the subsequent definitions in the relevant description.

As used herein, the term "$EC_{50}$", i.e., the half-maximal effective concentration, refers to the concentration of an antibody at which the induced response reaches 50% of the maximal response, representing the midpoint between the maximal response and baseline. The $EC_{50}$ can be measured by ELISA or FACS analysis or any other method known in the art.

The "$K_D$" refers to the equilibrium dissociation constant, which is derived from the ratio of the dissociation rate constant ($k_d$) to the association rate constant ($k_a$) (i.e., $k_d/k_a$), and is expressed in molar concentration (M). The $K_D$ value of an antibody may be determined using methods well established in the art. A preferred method for determining the $K_D$ of an antibody is surface plasmon resonance (SPR), and a biosensor system, such as the Biacore® surface plasmon resonance system, is preferably used for analysis.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to acquire the maximum alignment for the full length of sequences being compared.

The term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present disclosure is a human. The terms "patient" and "subject" are used interchangeably unless otherwise indicated. The "subjects in need" include those with a disease or condition, those at risk of developing a disease or condition, and those who may have a disease or condition and whose purpose is to prevent, delay, or alleviate the disease or condition.

As used herein, "about" means being within an acceptable error range determined by those of ordinary skill in the art for a specific value, which will depend in part on how the value is measured or determined, i.e., the limitation by the measurement system. For example, "about" may mean being within 1 or more than 1 standard deviation, as practiced in the art. Alternatively, "about" may mean a range of up to $\pm5\%$, for example, fluctuating within a specific numerical range given $\pm2\%$, $\pm1\%$, or $\pm0.5\%$. Where a specific value is given in the scope of the present disclosure, unless otherwise stated, "about" should be considered to mean being within an acceptable error range for that specific value. As used herein, unless otherwise stated, the values of the parameters or conditions in a step are all modified by "about" by default.

The terms "comprise", "comprises", "comprising", and equivalents thereof (e.g., contain, contains, containing, include, includes, and including) are to be construed as "including, but not limited to", meaning that additional unspecified elements, components, and steps may be included in addition to the enumerated elements, components, and steps.

As used herein, unless otherwise specified clearly in the context, singular terms encompass plural referents, and vice versa.

*Anti-ROR1 Antibody or Antigen-Binding Fragment Thereof*

The present disclosure provides an anti-ROR1 antibody or an antigen-binding fragment thereof, which comprises: an HCDR1 comprising the amino acid sequence set forth in SEQ ID NO: 1, 9, 17, or 25, an HCDR2 comprising the amino acid sequence set forth in SEQ ID NO: 2, 10, 18, 26, 49, or 50, an HCDR3 comprising the amino acid sequence set forth in SEQ ID NO: 3, 11, 19, or 27, an LCDR1 comprising the amino acid sequence set forth in SEQ ID NO: 4, 12, 20, or 28, an LCDR2 comprising the amino acid sequence set forth in SEQ ID NO: 5, 13, 21, or 29, and an LCDR3 comprising the amino acid sequence set forth in SEQ ID NO: 6, 14, 22, or 30.

In some embodiments, the anti-ROR1 antibody or the antigen-binding fragment thereof comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the anti-ROR1 antibody or the antigen-binding fragment thereof comprises: an HCDR1 comprising the amino acid sequence set forth in SEQ ID NO: 9, an HCDR2 comprising the amino acid sequence set forth in SEQ ID NO: 50, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14, wherein $X_1$ is selected from the group consisting of S and A, $X_2$ is selected from the group consisting of A and Q, and $X_3$ is selected from the group consisting of K and Q. In some specific embodiments, $X_1$ is S, $X_2$ is A, and $X_3$ is K. In some specific embodiments, $X_1$ is A, $X_2$ is Q, and $X_3$ is Q.

In some embodiments, the anti-ROR1 antibody or the antigen-binding fragment thereof comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody or the antigen-binding fragment thereof comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

The present disclosure also provides an anti-ROR1 antibody or an antigen-binding fragment thereof, which comprises: an HCDR1, an HCDR2, and an HCDR3 in a heavy chain variable region having the amino acid sequence set forth in SEQ ID NO: 7, 15, 23, 31, 51, 52, 56, 57, 58, 60, or 61, and an LCDR1, an LCDR2, and an LCDR3 in a light chain variable region having the amino acid sequence set forth in SEQ ID NO: 8, 16, 24, 32, 53, 54, 55, 59, 62, 63, or 64.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 53.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 54.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 55.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 53.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 54.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 55.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 56, and an LCDR1, an LCDR2, and an LCDR3 in a light chain variable region having the amino acid sequence set forth in SEQ ID NO: 59.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 57, and an LCDR1, an LCDR2, and an LCDR3 in a light chain variable region having the amino acid sequence set forth in SEQ ID NO: 59.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 58, and an LCDR1, an LCDR2, and an LCDR3 in a light chain variable region having the amino acid sequence set forth in SEQ ID NO: 59.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 62.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 63.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 64.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 62.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 63.

In some embodiments, the anti-ROR1 antibody or the antigen-binding fragment thereof comprises: an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 64.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 15, 23, 31, 51, 52, 56, 57, 58, 60, or 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8, 16, 24, 32, 53, 54, 55, 59, 62, 63, or 64.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. Further, in some of such embodiments, the anti-ROR1 antibody or the antigen-binding fragment thereof further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24. Further, in some of such embodiments, the anti-ROR1 antibody or the antigen-binding fragment thereof further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55. Further, in some of such embodiments, the anti-ROR1 antibody or the antigen-binding fragment thereof further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 56. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 56, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. Further, in some of such embodiments, the anti-ROR1 antibody or the antigen-binding fragment thereof further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 57. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 57, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. Further, in some of such embodiments, the anti-ROR1 antibody or the antigen-binding fragment thereof further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 58. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 58, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59. Further, in some of such embodiments, the anti-ROR1 antibody or the antigen-binding fragment thereof further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61. In some embodiments, the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64. In some embodiments, the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64. Further, in some of such embodiments, the anti-ROR1 antibody or the antigen-binding fragment thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody or the antigen-binding fragment thereof comprises: a heavy chain variable region comprising the amino acid sequence set forth in SEQ ID NO: 7 and a light chain variable region comprising the amino acid sequence set forth in SEQ ID NO: 8.

In some embodiments, the anti-ROR1 antibody or the antigen-binding fragment thereof comprises: a heavy chain variable region comprising the amino acid sequence set forth in SEQ ID NO: 15 and a light chain variable region comprising the amino acid sequence set forth in SEQ ID NO: 16.

In some embodiments, the anti-ROR1 antibody or the antigen-binding fragment thereof comprises: a heavy chain variable region comprising the amino acid sequence set forth in SEQ ID NO: 23 and a light chain variable region comprising the amino acid sequence set forth in SEQ ID NO: 24.

In some embodiments, the anti-ROR1 antibody or the antigen-binding fragment thereof comprises: a heavy chain variable region comprising the amino acid sequence set forth in SEQ ID NO: 31 and a light chain variable region comprising the amino acid sequence set forth in SEQ ID NO: 32.

In some embodiments, the anti-ROR1 antibody or the antigen-binding fragment thereof comprises: a heavy chain variable region comprising the amino acid sequence set forth in SEQ ID NO: 51 and a light chain variable region comprising the amino acid sequence set forth in SEQ ID NO: 53.

In some embodiments, the anti-ROR1 antibody or the antigen-binding fragment thereof comprises: a heavy chain variable region comprising the amino acid sequence set forth in SEQ ID NO: 51 and a light chain variable region comprising the amino acid sequence set forth in SEQ ID NO: 54.

In some embodiments, the anti-ROR1 antibody or the antigen-binding fragment thereof comprises: a heavy chain variable region comprising the amino acid sequence set forth in SEQ ID NO: 51 and a light chain variable region comprising the amino acid sequence set forth in SEQ ID NO: 55.

In some embodiments, the anti-ROR1 antibody or the antigen-binding fragment thereof comprises: a heavy chain variable region comprising the amino acid sequence set forth in SEQ ID NO: 52 and a light chain variable region comprising the amino acid sequence set forth in SEQ ID NO: 53.

In some embodiments, the anti-ROR1 antibody or the antigen-binding fragment thereof comprises: a heavy chain variable region comprising the amino acid sequence set forth in SEQ ID NO: 52 and a light chain variable region comprising the amino acid sequence set forth in SEQ ID NO: 54.

In some embodiments, the anti-ROR1 antibody or the antigen-binding fragment thereof comprises: a heavy chain variable region comprising the amino acid sequence set forth in SEQ ID NO: 52 and a light chain variable region comprising the amino acid sequence set forth in SEQ ID NO: 55.

In some embodiments, the anti-ROR1 antibody or the antigen-binding fragment thereof comprises: a heavy chain variable region comprising the amino acid sequence set forth in SEQ ID NO: 56 and a light chain variable region comprising the amino acid sequence set forth in SEQ ID NO: 59.

In some embodiments, the anti-ROR1 antibody or the antigen-binding fragment thereof comprises: a heavy chain variable region comprising the amino acid sequence set forth in SEQ ID NO: 57 and a light chain variable region comprising the amino acid sequence set forth in SEQ ID NO: 59.

In some embodiments, the anti-ROR1 antibody or the antigen-binding fragment thereof comprises: a heavy chain variable region comprising the amino acid sequence set forth in SEQ ID NO: 58 and a light chain variable region comprising the amino acid sequence set forth in SEQ ID NO: 59.

In some embodiments, the anti-ROR1 antibody or the antigen-binding fragment thereof comprises: a heavy chain variable region comprising the amino acid sequence set forth in SEQ ID NO: 60 and a light chain variable region comprising the amino acid sequence set forth in SEQ ID NO: 62.

In some embodiments, the anti-ROR1 antibody or the antigen-binding fragment thereof comprises: a heavy chain variable region comprising the amino acid sequence set forth in SEQ ID NO: 60 and a light chain variable region comprising the amino acid sequence set forth in SEQ ID NO: 63.

In some embodiments, the anti-ROR1 antibody or the antigen-binding fragment thereof comprises: a heavy chain variable region comprising the amino acid sequence set forth in SEQ ID NO: 60 and a light chain variable region comprising the amino acid sequence set forth in SEQ ID NO: 64.

In some embodiments, the anti-ROR1 antibody or the antigen-binding fragment thereof comprises: a heavy chain variable region comprising the amino acid sequence set forth in SEQ ID NO: 61 and a light chain variable region comprising the amino acid sequence set forth in SEQ ID NO: 62.

In some embodiments, the anti-ROR1 antibody or the antigen-binding fragment thereof comprises: a heavy chain variable region comprising the amino acid sequence set forth in SEQ ID NO: 61 and a light chain variable region comprising the amino acid sequence set forth in SEQ ID NO: 63.

In some embodiments, the anti-ROR1 antibody or the antigen-binding fragment thereof comprises: a heavy chain variable region comprising the amino acid sequence set forth in SEQ ID NO: 61 and a light chain variable region comprising the amino acid sequence set forth in SEQ ID NO: 64.

In the present disclosure, the antigen-binding fragment is a Fab fragment, a F(ab')2 fragment, an Fd fragment, an Fv fragment, an isolated CDR region, or an scFv. In the present disclosure, the anti-ROR1 antibody or the antigen-binding fragment thereof is murine, chimeric, or humanized. Humanization may reduce immunogenicity. Therefore, in some embodiments, the anti-ROR1 antibody or the antigen-binding fragment thereof is humanized.

In some embodiments, the anti-ROR1 antibody or the antigen-binding fragment thereof of the present disclosure may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, IgG4, or other types of immunoglobulins. In some embodiments, the light chain constant region is derived from a human immunoglobulin light chain, for example, a κ light chain or a λ light chain of a human immunoglobulin. In some embodiments, the anti-ROR1 antibody is of the IgG1, IgG2, IgG3, or IgG4 isotype. In some embodiments, the constant region may comprise any modification disclosed herein or well-known in the art, such as an insertion, a deletion, a substitution, or a chemical modification of an amino acid. The C-terminal lysine (Lys at position 447 according to EU numbering) of the heavy chain constant region may be present or deleted. In some embodiments, the constant region comprises a mutation that alters the effector function. In some embodiments, any amino acid residue of the constant region may be substituted by an amino acid residue of any allotype.

In some specific embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33, 37, 41, 45, 65, 67, 75, 77, 79, 83, or 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35, 39, 43, 47, 69, 71, 73, 81, 87, 89, or 91.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35. Further, in some of such embodiments, the anti-ROR1 antibody further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 43. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 43. Further, in some of such embodiments, the anti-ROR1 antibody further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 45. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 47. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 45, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 47. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73. Further, in some of such embodiments, the anti-ROR1 antibody further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. Further, in some of such embodiments, the anti-ROR1 antibody further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. Further, in some of such embodiments, the anti-ROR1 antibody further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81. Further, in some of such embodiments, the anti-ROR1 antibody further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85. In some embodiments, the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91. In some embodiments, the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91. Further, in some of such embodiments, the anti-ROR1 antibody further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the anti-ROR1 antibody comprises: a heavy chain comprising the amino acid sequence set forth in SEQ ID NO: 33 and a light chain comprising the amino acid sequence set forth in SEQ ID NO: 35.

In some embodiments, the anti-ROR1 antibody comprises: a heavy chain comprising the amino acid sequence set forth in SEQ ID NO: 37 and a light chain comprising the amino acid sequence set forth in SEQ ID NO: 39.

In some embodiments, the anti-ROR1 antibody comprises: a heavy chain comprising the amino acid sequence set forth in SEQ ID NO: 41 and a light chain comprising the amino acid sequence set forth in SEQ ID NO: 43.

In some embodiments, the anti-ROR1 antibody comprises: a heavy chain comprising the amino acid sequence set forth in SEQ ID NO: 45 and a light chain comprising the amino acid sequence set forth in SEQ ID NO: 47.

In some embodiments, the anti-ROR1 antibody comprises: a heavy chain comprising the amino acid sequence set forth in SEQ ID NO: 65 and a light chain comprising the amino acid sequence set forth in SEQ ID NO: 69.

In some embodiments, the anti-ROR1 antibody comprises: a heavy chain comprising the amino acid sequence set forth in SEQ ID NO: 65 and a light chain comprising the amino acid sequence set forth in SEQ ID NO: 71.

In some embodiments, the anti-ROR1 antibody comprises: a heavy chain comprising the amino acid sequence set forth in SEQ ID NO: 65 and a light chain comprising the amino acid sequence set forth in SEQ ID NO: 73.

In some embodiments, the anti-ROR1 antibody comprises: a heavy chain comprising the amino acid sequence set forth in SEQ ID NO: 67 and a light chain comprising the amino acid sequence set forth in SEQ ID NO: 69.

In some embodiments, the anti-ROR1 antibody comprises: a heavy chain comprising the amino acid sequence set forth in SEQ ID NO: 67 and a light chain comprising the amino acid sequence set forth in SEQ ID NO: 71.

In some embodiments, the anti-ROR1 antibody comprises: a heavy chain comprising the amino acid sequence set forth in SEQ ID NO: 67 and a light chain comprising the amino acid sequence set forth in SEQ ID NO: 73.

In some embodiments, the anti-ROR1 antibody comprises: a heavy chain comprising the amino acid sequence set forth in SEQ ID NO: 75 and a light chain comprising the amino acid sequence set forth in SEQ ID NO: 81.

In some embodiments, the anti-ROR1 antibody comprises: a heavy chain comprising the amino acid sequence set forth in SEQ ID NO: 77 and a light chain comprising the amino acid sequence set forth in SEQ ID NO: 81.

In some embodiments, the anti-ROR1 antibody comprises: a heavy chain comprising the amino acid sequence set forth in SEQ ID NO: 79 and a light chain comprising the amino acid sequence set forth in SEQ ID NO: 81.

In some embodiments, the anti-ROR1 antibody comprises: a heavy chain comprising the amino acid sequence set forth in SEQ ID NO: 83 and a light chain comprising the amino acid sequence set forth in SEQ ID NO: 87.

In some embodiments, the anti-ROR1 antibody comprises: a heavy chain comprising the amino acid sequence set forth in SEQ ID NO: 83 and a light chain comprising the amino acid sequence set forth in SEQ ID NO: 89.

In some embodiments, the anti-ROR1 antibody comprises: a heavy chain comprising the amino acid sequence set forth in SEQ ID NO: 83 and a light chain comprising the amino acid sequence set forth in SEQ ID NO: 91.

In some embodiments, the anti-ROR1 antibody comprises: a heavy chain comprising the amino acid sequence set forth in SEQ ID NO: 85 and a light chain comprising the amino acid sequence set forth in SEQ ID NO: 87.

In some embodiments, the anti-ROR1 antibody comprises: a heavy chain comprising the amino acid sequence set forth in SEQ ID NO: 85 and a light chain comprising the amino acid sequence set forth in SEQ ID NO: 89.

In some embodiments, the anti-ROR1 antibody comprises: a heavy chain comprising the amino acid sequence set forth in SEQ ID NO: 85 and a light chain comprising the amino acid sequence set forth in SEQ ID NO: 91.

In some embodiments, the anti-ROR1 antibody comprises a heavy chain having the amino acid sequence set forth in SEQ ID NO: 33 and a light chain having the amino acid sequence set forth in SEQ ID NO: 35.

In some embodiments, the anti-ROR1 antibody comprises a heavy chain having the amino acid sequence set forth in SEQ ID NO: 37 and a light chain having the amino acid sequence set forth in SEQ ID NO: 39.

In some embodiments, the anti-ROR1 antibody comprises a heavy chain having the amino acid sequence set forth in SEQ ID NO: 41 and a light chain having the amino acid sequence set forth in SEQ ID NO: 43.

In some embodiments, the anti-ROR1 antibody comprises a heavy chain having the amino acid sequence set forth in SEQ ID NO: 45 and a light chain having the amino acid sequence set forth in SEQ ID NO: 47.

In some embodiments, the anti-ROR1 antibody comprises a heavy chain having the amino acid sequence set forth in SEQ ID NO: 65 and a light chain having the amino acid sequence set forth in SEQ ID NO: 69.

In some embodiments, the anti-ROR1 antibody comprises a heavy chain having the amino acid sequence set forth in SEQ ID NO: 65 and a light chain having the amino acid sequence set forth in SEQ ID NO: 71.

In some embodiments, the anti-ROR1 antibody comprises a heavy chain having the amino acid sequence set forth in SEQ ID NO: 65 and a light chain having the amino acid sequence set forth in SEQ ID NO: 73.

In some embodiments, the anti-ROR1 antibody comprises a heavy chain having the amino acid sequence set forth in SEQ ID NO: 67 and a light chain having the amino acid sequence set forth in SEQ ID NO: 69.

In some embodiments, the anti-ROR1 antibody comprises a heavy chain having the amino acid sequence set forth in SEQ ID NO: 67 and a light chain having the amino acid sequence set forth in SEQ ID NO: 71.

In some embodiments, the anti-ROR1 antibody comprises a heavy chain having the amino acid sequence set forth in SEQ ID NO: 67 and a light chain having the amino acid sequence set forth in SEQ ID NO: 73.

In some embodiments, the anti-ROR1 antibody comprises a heavy chain having the amino acid sequence set forth in SEQ ID NO: 75 and a light chain having the amino acid sequence set forth in SEQ ID NO: 81.

In some embodiments, the anti-ROR1 antibody comprises a heavy chain having the amino acid sequence set forth in SEQ ID NO: 77 and a light chain having the amino acid sequence set forth in SEQ ID NO: 81.

In some embodiments, the anti-ROR1 antibody comprises a heavy chain having the amino acid sequence set forth in SEQ ID NO: 79 and a light chain having the amino acid sequence set forth in SEQ ID NO: 81.

In some embodiments, the anti-ROR1 antibody comprises a heavy chain having the amino acid sequence set forth in SEQ ID NO: 83 and a light chain having the amino acid sequence set forth in SEQ ID NO: 87.

In some embodiments, the anti-ROR1 antibody comprises a heavy chain having the amino acid sequence set forth in SEQ ID NO: 83 and a light chain having the amino acid sequence set forth in SEQ ID NO: 89.

In some embodiments, the anti-ROR1 antibody comprises a heavy chain having the amino acid sequence set forth in SEQ ID NO: 83 and a light chain having the amino acid sequence set forth in SEQ ID NO: 91.

In some embodiments, the anti-ROR1 antibody comprises a heavy chain having the amino acid sequence set forth in SEQ ID NO: 85 and a light chain having the amino acid sequence set forth in SEQ ID NO: 87.

In some embodiments, the anti-ROR1 antibody comprises a heavy chain having the amino acid sequence set forth in SEQ ID NO: 85 and a light chain having the amino acid sequence set forth in SEQ ID NO: 89.

In some embodiments, the anti-ROR1 antibody comprises a heavy chain having the amino acid sequence set forth in SEQ ID NO: 85 and a light chain having the amino acid sequence set forth in SEQ ID NO: 91.

In some embodiments, the amino acid sequence of the heavy chain of the anti-ROR1 antibody is set forth in SEQ ID NO: 33, and the amino acid sequence of the light chain is set forth in SEQ ID NO: 35.

In some embodiments, the amino acid sequence of the heavy chain of the anti-ROR1 antibody is set forth in SEQ ID NO: 37, and the amino acid sequence of the light chain is set forth in SEQ ID NO: 39.

In some embodiments, the amino acid sequence of the heavy chain of the anti-ROR1 antibody is set forth in SEQ ID NO: 41, and the amino acid sequence of the light chain is set forth in SEQ ID NO: 43.

In some embodiments, the amino acid sequence of the heavy chain of the anti-ROR1 antibody is set forth in SEQ ID NO: 45, and the amino acid sequence of the light chain is set forth in SEQ ID NO: 47.

In some embodiments, the amino acid sequence of the heavy chain of the anti-ROR1 antibody is set forth in SEQ ID NO: 65, and the amino acid sequence of the light chain is set forth in SEQ ID NO: 69.

In some embodiments, the amino acid sequence of the heavy chain of the anti-ROR1 antibody is set forth in SEQ ID NO: 65, and the amino acid sequence of the light chain is set forth in SEQ ID NO: 71.

In some embodiments, the amino acid sequence of the heavy chain of the anti-ROR1 antibody is set forth in SEQ ID NO: 65, and the amino acid sequence of the light chain is set forth in SEQ ID NO: 73.

In some embodiments, the amino acid sequence of the heavy chain of the anti-ROR1 antibody is set forth in SEQ ID NO: 67, and the amino acid sequence of the light chain is set forth in SEQ ID NO: 69.

In some embodiments, the amino acid sequence of the heavy chain of the anti-ROR1 antibody is set forth in SEQ ID NO: 67, and the amino acid sequence of the light chain is set forth in SEQ ID NO: 71.

In some embodiments, the amino acid sequence of the heavy chain of the anti-ROR1 antibody is set forth in SEQ ID NO: 67, and the amino acid sequence of the light chain is set forth in SEQ ID NO: 73.

In some embodiments, the amino acid sequence of the heavy chain of the anti-ROR1 antibody is set forth in SEQ ID NO: 75, and the amino acid sequence of the light chain is set forth in SEQ ID NO: 81.

In some embodiments, the amino acid sequence of the heavy chain of the anti-ROR1 antibody is set forth in SEQ ID NO: 77, and the amino acid sequence of the light chain is set forth in SEQ ID NO: 81.

In some embodiments, the amino acid sequence of the heavy chain of the anti-ROR1 antibody is set forth in SEQ ID NO: 79, and the amino acid sequence of the light chain is set forth in SEQ ID NO: 81.

In some embodiments, the amino acid sequence of the heavy chain of the anti-ROR1 antibody is set forth in SEQ ID NO: 83, and the amino acid sequence of the light chain is set forth in SEQ ID NO: 87.

In some embodiments, the amino acid sequence of the heavy chain of the anti-ROR1 antibody is set forth in SEQ ID NO: 83, and the amino acid sequence of the light chain is set forth in SEQ ID NO: 89.

In some embodiments, the amino acid sequence of the heavy chain of the anti-ROR1 antibody is set forth in SEQ ID NO: 83, and the amino acid sequence of the light chain is set forth in SEQ ID NO: 91.

In some embodiments, the amino acid sequence of the heavy chain of the anti-ROR1 antibody is set forth in SEQ ID NO: 85, and the amino acid sequence of the light chain is set forth in SEQ ID NO: 87.

In some embodiments, the amino acid sequence of the heavy chain of the anti-ROR1 antibody is set forth in SEQ ID NO: 85, and the amino acid sequence of the light chain is set forth in SEQ ID NO: 89.

In some embodiments, the amino acid sequence of the heavy chain of the anti-ROR1 antibody is set forth in SEQ ID NO: 85, and the amino acid sequence of the light chain is set forth in SEQ ID NO: 91.

It should be understood that the anti-ROR1 antibodies referred to herein also include anti-ROR1 antibodies with deletion of lysine (Lys at position 447 according to EU numbering) at the C-terminus of the heavy chain.

Tables S1 and S2 provide amino acid sequence information of CDRs and variable regions of some exemplary anti-ROR1 antibodies or antigen-binding fragments thereof (e.g., chimeric antibodies ch3G9B1, ch29H5D8, ch135A2B10, and ch137G11D10, as well as humanized antibodies hz29H5D8-1.1, hz29H5D8-1.2, hz29H5D8-2.1, hz29H5D8-2.2, hz29H5D8-3.1, hz29H5D8-3.2, hz135A2B10-1.1, hz135A2B10-1.2, hz135A2B10-1.3, hz137G11D10-1.1, hz137G11D10-1.2, hz137G11D10-2.1, hz137G11D10-2.2, hz137G11D10-3.1, and hz137G11D10-3.2).

Table S1. Amino acid sequences of CDRs and variable regions (SEQ ID NO:)

| Name | HCDR1 | HCDR2 | HCDR3 | VH | LCDR1 | LCDR2 | LCDR3 | VL |
|---|---|---|---|---|---|---|---|---|
| m3G9B1 | 1 | 2 | 3 | 7 | 4 | 5 | 6 | 8 |
| ch29H5D8 | 9 | 10 | 11 | 15 | 12 | 13 | 14 | 16 |
| hz29H5D8-1.1 | | 49 | | 51 | | | | 53 |
| hz29H5D8-2.1 | | | | | | | | 54 |
| hz29H5D8-3.1 | | | | | | | | 55 |
| hz29H5D8-1.2 | | 10 | | 52 | | | | 53 |
| hz29H5D8-2.2 | | | | | | | | 54 |
| hz29H5D8-3.2 | | | | | | | | 55 |
| ch135A2B10 | 17 | 18 | 19 | 23 | 20 | 21 | 22 | 24 |
| hz135A2B10-1.1 | | | | 56 | | | | |
| hz135A2B10-1.2 | | | | 57 | | | | 59 |
| hz135A2B10-1.3 | | | | 58 | | | | |
| ch137G11D10 | 25 | 26 | 27 | 31 | 28 | 29 | 30 | 32 |
| hz137G11D10-1.1 | | | | 60 | | | | 62 |
| hz137G11D10-2.1 | | | | | | | | 63 |
| hz137G11D10-3.1 | | | | | | | | 64 |
| hz137G11D10-1.2 | | | | 61 | | | | 62 |
| hz137G11D10-2.2 | | | | | | | | 63 |
| hz137G11D10-3.2 | | | | | | | | 64 |

Any one of the anti-ROR1 antibodies or the antigen-binding fragments thereof provided in the present disclosure exhibits good antigen-binding activity. Any one of the anti-ROR1 antibodies or the antigen-binding fragments thereof of the present disclosure can bind to human ROR1 or/and monkey ROR1. In some embodiments, any one of the anti-ROR1 antibodies or the antigen-binding fragments thereof of the present disclosure can bind to human ROR1 with the following $K_D$: 5E-08 M or less, 2.71E-08 M or less, 2E-08 M or less, 1E-08 M or less, 5E-09 M or less, 2E-09 M or less, 1.17E-09 M or less, 1E-09 M or less, 6.69E-10 M or less, 6.1E-10 M or less, 5.89E-10 M or less, 5.86E-10 M or less, 5.75E-10 M or less, 5.69E-10 M or less, 5.27E-10 M or less, 5E-10 M or less, 3.43E-10 M or less, 2.89E-10 M or less, 1.97E-10 M or less, 1.72E-10 M or less, 1.6E-10 M or less, 1.45E-10 M or less, 1.12E-10 M or less, or 1.05E-10 M or less, as measured by surface plasmon resonance (SPR). In some embodiments, any one of the anti-ROR1 antibodies or the antigen-binding fragments thereof of the present disclosure can bind to monkey ROR1 with the following $K_D$: 5E-08 M or less, 2.71E-08 M or less, 2E-08 M or less, 1E-08 M or less, 5E-09 M or less, 2E-09 M or less, 1.17E-09 M or less, 1E-09 M or less, 6.69E-10 M or less, 6.1E-10 M or less, 5.89E-10 M or less, 5.86E-10 M or less, 5.75E-10 M or less, 5.69E-10 M or less, 5.27E-10 M or less, 5E-10 M or less, 3.43E-10 M or less, 2.89E-10 M or less, 1.97E-10 M or less, 1.72E-10 M or less, 1.6E-10 M or less, 1.45E-10 M or less, 1.12E-10 M or less, or 1.05E-10 M or less, as measured by surface plasmon resonance (SPR).

Any one of the anti-ROR1 antibodies or the antigen-binding fragments thereof provided in the present disclosure is capable of effectively exerting an internalization effect in cells expressing ROR1.

Any one of the anti-ROR1 antibodies or the antigen-binding fragments thereof provided in the present disclosure is capable of inhibiting the Wnt5a-stimulated non-classical Wnt signaling pathway in cells expressing ROR1. In some embodiments, the anti-ROR1 antibody or the antigen-binding fragment thereof is capable of inhibiting the Wnt5a-stimulated non-classical Wnt signaling pathway at 20 nM or less, 10 nM or less, 5 nM or less, or 2.5 nM or less. In some embodiments, any one of the anti-ROR1 antibodies or the antigen-binding fragments thereof of the present disclosure has an inhibition rate of 10% or higher, 15% or higher, or 20% or higher against the Wnt5a-stimulated non-classical Wnt signaling pathway at a concentration of 20 nM.

Any one of the anti-ROR1 antibodies or the antigen-binding fragments thereof provided in the present disclosure is capable of exerting an ADCC effect on cells expressing ROR1.

Any one of the anti-ROR1 antibodies or the antigen-binding fragments thereof provided in the present disclosure can induce the regression of tumor growth *in vivo.* In some embodiments, the tumor is a tumor expressing ROR1. Any one of the anti-ROR1 antibodies or the antigen-binding fragments thereof provided in the present disclosure can delay tumor growth *in vivo.* In some embodiments, the tumor is a tumor expressing ROR1.

Any one of the anti-ROR1 antibodies or the antigen-binding fragments thereof of the present disclosure exhibits good safety.

In some embodiments, the anti-ROR1 antibody or the antigen-binding fragment thereof is a monoclonal antibody. In some embodiments, the anti-ROR1 antibody or the antigen-binding fragment thereof is a monospecific antibody. In some embodiments, the anti-ROR1 antibody or the antigen-binding fragment thereof is a multispecific antibody (e.g., a bispecific antibody or a trispecific antibody).

*Isolated Nucleic Acid*

The present disclosure provides an isolated nucleic acid, which comprises a nucleotide sequence encoding the anti-ROR1 antibody or the antigen-binding fragment thereof described in the present disclosure. Some nucleotide sequences encoding the anti-ROR1 antibody or the antigen-binding fragment thereof are illustratively listed in the sequence listing.

*Vector*

The present disclosure provides a vector comprising the nucleic acid. In some embodiments, the vector is a cloning vector. In some other embodiments, the vector is an expression vector. As a specific example, the expression vector is pcDNA3.1(+). The expression vector is optionally any expression vector capable of expressing the anti-ROR1 antibody or the antigen-binding fragment thereof described in the present disclosure.

*Host Cell*

The present disclosure provides a host cell comprising the nucleic acid of the present disclosure or the vector of the present disclosure. In some embodiments, the host cell is an appropriate host cell for cloning or expressing the anti-ROR1 antibody or the antigen-binding fragment thereof. In some embodiments, the host cell is a prokaryotic cell. In some other embodiments, the host cell is a eukaryotic cell. In some embodiments, the host cell is selected from the group consisting of a yeast cell, a mammalian cell, and other cells suitable for preparing the anti-ROR1 antibody or the antigen-binding fragment thereof. Mammalian cells are, for example, Chinese hamster ovary (CHO) cells or CHO-S cells.

*Fusion Protein*

In some embodiments, the present disclosure provides a fusion protein, which comprises the anti-ROR1 antibody or the antigen-binding fragment thereof of the present disclosure.

*Antibody-Drug Conjugate (ADC)*

The present disclosure provides an antibody-drug conjugate, which comprises the anti-ROR1 antibody or the antigen-binding fragment thereof of the present disclosure. In some embodiments, the antibody-drug conjugate comprises the anti-ROR1 antibody or the antigen-binding fragment thereof and a cytotoxic drug.

*Chimeric Antigen Receptor (CAR)*

The present disclosure also provides an anti-ROR1 chimeric antigen receptor (CAR), which comprises the anti-ROR1 antibody or the antigen-binding fragment thereof of the present disclosure.

The anti-ROR1 CAR may comprise (a) an extracellular antigen-binding domain that binds to ROR1; (b) a transmembrane domain; (c) an intracellular signaling domain.

The present disclosure also provides an engineered immune effector cell, which comprises the CAR of the present disclosure. In some embodiments, the immune effector cell is a T cell, an NK cell, a peripheral blood mononuclear cell (PBMC), a hematopoietic stem cell, a pluripotent stem cell, or an embryonic stem cell.

*Pharmaceutical Composition*

The present disclosure provides a pharmaceutical composition, and the pharmaceutical composition comprises the anti-ROR1 antibody or the antigen-binding fragment thereof, the fusion protein, the antibody-drug conjugate, or the engineered immune effector cell of the present disclosure, and further comprises one or more pharmaceutically acceptable excipients. The pharmaceutically acceptable excipient includes, for example, an excipient, a diluent, an encapsulating material, a filler, a buffering agent, or other reagents.

*Method for Preparing Anti-ROR1 Antibody or Antigen-Binding Fragment Thereof*

In some embodiments, the present disclosure provides a method for preparing the anti-ROR1 antibody or the antigen-binding fragment thereof, which comprises: culturing the host cell to express the anti-ROR1 antibody or the antigen-binding fragment thereof, and isolating and purifying the anti-ROR1 antibody or the antigen-binding fragment thereof in the system. To produce the anti-ROR1 antibody or the antigen-binding fragment thereof, the nucleic acid encoding the anti-ROR1 antibody or the antigen-binding fragment thereof is isolated and inserted into one or more vectors for further cloning or/and expression in a host cell. The nucleic acid can be obtained using various methods known in the art, such as gene splicing and chemical synthesis.

The purity of the anti-ROR1 antibody or the antigen-binding fragment thereof can be determined by any one of various well-known analytical methods, such as gel electrophoresis, high-performance liquid chromatography, and size-exclusion chromatography.

The physicochemical properties or/and biological activity of the anti-ROR1 antibody or the antigen-binding fragment thereof of the present disclosure can be identified, screened, or characterized by various assay methods known in the art.

*Use*

The present disclosure provides use of the anti-ROR1 antibody or the antigen-binding fragment thereof of the present disclosure. In some specific embodiments, the anti-ROR1 antibody used may be ch3G9B1, ch29H5D8, ch135A2B10, ch137G11D10, hz29H5D8-1.1, hz29H5D8-1.2, hz29H5D8-2.1, hz29H5D8-2.2, hz29H5D8-3.1, hz29H5D8-3.2, hz135A2B10-1.1, hz135A2B10-1.2, hz135A2B10-1.3, hz137G11D10-1.1, hz137G11D10-1.2, hz137G11D10-2.1, hz137G11D10-2.2, hz137G11D10-3.1, or/and hz137G11D10-3.2.

The present disclosure provides use of the anti-ROR1 antibody or the antigen-binding fragment thereof, the fusion protein, the antibody-drug conjugate, the engineered immune effector cell, or the pharmaceutical composition for preparing a medicament for treating a disease expressing ROR1.

The present disclosure provides a method for treating a disease expressing ROR1, which comprises administering to a subject in need the anti-ROR1 antibody or the antigen-binding fragment thereof, the fusion protein, the antibody-drug conjugate, the engineered immune effector cell, or the pharmaceutical composition. The present disclosure provides a method for treating a disease expressing ROR1, which comprises administering to a subject in need a therapeutically effective amount of the anti-ROR1 antibody or the antigen-binding fragment thereof, the fusion protein, the antibody-drug conjugate, the engineered immune effector cell, or the pharmaceutical composition. In some embodiments, the disease is a tumor.

In some embodiments, the method comprises contacting a tumor cell with the anti-ROR1 antibody or the antigen-binding fragment thereof, the fusion protein, the antibody-drug conjugate, the engineered immune effector cell, or the pharmaceutical composition, thereby killing the tumor cell or inhibiting the growth of the tumor cell.

In some embodiments, the tumor is a non-solid tumor. In some embodiments, the tumor is a hematological tumor. In some embodiments, the tumor is a solid tumor. In some embodiments, the tumor is leukemia, lymphoma, multiple myeloma, breast cancer, ovarian cancer, ovarian teratoma, colon cancer, lung cancer (including small cell lung cancer and non-small cell lung cancer), skin cancer, pancreatic cancer, testicular cancer, bladder cancer, uterine cancer, prostate cancer, or adrenal cancer. In some embodiments, the lymphoma is mantle cell lymphoma (MCL).

*Kit*

The present disclosure provides a kit, which comprises the anti-ROR1 antibody or the antigen-binding fragment thereof, the fusion protein, the antibody-drug conjugate, the engineered immune effector cell, or the pharmaceutical composition of the present disclosure. In some embodiments, the kit may be used to implement the use of the anti-ROR1 antibody or the antigen-binding fragment thereof, the fusion protein, the antibody-drug conjugate, the engineered immune effector cell, or the pharmaceutical composition provided in the present disclosure, or other uses. In some embodiments, the kit may comprise the anti-ROR1 antibody or the antigen-binding fragment thereof, the fusion protein, the antibody-drug conjugate, the engineered immune effector cell, or the pharmaceutical composition of the present disclosure, and a reagent for detecting the presence of ROR1 in a biological sample. The kit may further comprise an instruction for use. The kit may further comprise other materials as required from commercial and user perspectives, for example, other buffers, diluents, needles, syringes, and the like.

## DETAILED DESCRIPTION

The present disclosure also provides the following specific embodiments, which, however, are not intended to limit the protection scope of the present disclosure:

Embodiment 1. An anti-ROR1 antibody or an antigen-binding fragment thereof, comprising: an HCDR1 comprising the amino acid sequence set forth in SEQ ID NO: 1, 9, 17, or 25, an HCDR2 comprising the amino acid sequence set forth in SEQ ID NO: 2, 10, 18, 26, 49, or 50, an HCDR3 comprising the amino acid sequence set forth in SEQ ID NO: 3, 11, 19, or 27, an LCDR1 comprising the amino acid sequence set forth in SEQ ID NO: 4, 12, 20, or 28, an LCDR2 comprising the amino acid sequence set forth in SEQ ID NO: 5, 13, 21, or 29, and an LCDR3 comprising the amino acid sequence set forth in SEQ ID NO: 6, 14, 22, or 30.
Embodiment 2. The anti-ROR1 antibody or the antigen-binding fragment thereof according to embodiment 1, wherein the anti-ROR1 antibody or the antigen-binding fragment thereof comprises:

(1) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(2) an HCDR1 comprising the amino acid sequence set forth in SEQ ID NO: 9, an HCDR2 comprising the amino acid sequence set forth in SEQ ID NO: 50, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(3) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(4) an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(5) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22; or

(6) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

Embodiment 3. An anti-ROR1 antibody or an antigen-binding fragment thereof, comprising: an HCDR1, an HCDR2, and an HCDR3 in a heavy chain variable region having the amino acid sequence set forth in SEQ ID NO: 7, 15, 23, 31, 51, 52, 56, 57, 58, 60, or 61, and an LCDR1, an LCDR2, and an LCDR3 in a light chain variable region having the amino acid sequence set forth in SEQ ID NO: 8, 16, 24, 32, 53, 54, 55, 59, 62, 63, or 64.

Embodiment 4. The anti-ROR1 antibody or the antigen-binding fragment thereof according to embodiment 3, wherein the anti-ROR1 antibody or the antigen-binding fragment thereof comprises:

(1) an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8;

(2) an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16;

(3) an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24;

(4) an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32;

(5) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 53;

(6) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 54;

(7) an HCDR1, an HCDR2, and an HCDR3 in a heavy chain variable region having the amino acid sequence set forth in SEQ ID NO: 51, and an LCDR1, an LCDR2, and an LCDR3 in a light chain variable region having the amino acid sequence set forth in SEQ ID NO: 55;

(8) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 53;

(9) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 54;

(10) an HCDR1, an HCDR2, and an HCDR3 in a heavy chain variable region having the amino acid sequence set forth in SEQ ID NO: 52, and an LCDR1, an LCDR2, and an LCDR3 in a light chain variable region having the amino acid sequence set forth in SEQ ID NO: 55;

(11) an HCDR1, an HCDR2, and an HCDR3 in a heavy chain variable region having the amino acid sequence set forth in SEQ ID NO: 56, and an LCDR1, an LCDR2, and an LCDR3 in a light chain variable region having the amino acid sequence set forth in SEQ ID NO: 59;

(12) an HCDR1, an HCDR2, and an HCDR3 in a heavy chain variable region having the amino acid sequence set forth in SEQ ID NO: 57, and an LCDR1, an LCDR2, and an LCDR3 in a light chain variable region having the amino acid sequence set forth in SEQ ID NO: 59;

(13) an HCDR1, an HCDR2, and an HCDR3 in a heavy chain variable region having the amino acid sequence set forth in SEQ ID NO: 58, and an LCDR1, an LCDR2, and an LCDR3 in a light chain variable region having the amino acid sequence set forth in SEQ ID NO: 59;

(14) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 62;

(15) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 63;

(16) an HCDR1, an HCDR2, and an HCDR3 in a heavy chain variable region having the amino acid sequence set forth in SEQ ID NO: 60, and an LCDR1, an LCDR2, and an LCDR3 in a light chain variable region having the amino acid sequence set forth in SEQ ID NO: 64;

(17) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 62;

(18) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 63; or

(19) an HCDR1, an HCDR2, and an HCDR3 in a heavy chain variable region having the amino acid sequence set forth in SEQ ID NO: 61, and an LCDR1, an LCDR2, and an LCDR3 in a light chain variable region having the amino acid sequence set forth in SEQ ID NO: 64.

Embodiment 5. The anti-ROR1 antibody or the antigen-binding fragment thereof according to any one of embodiments 1-4, wherein the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 15, 23, 31, 51, 52, 56, 57, 58, 60, or 61.

Embodiment 6. The anti-ROR1 antibody or the antigen-binding fragment thereof according to any one of embodiments 1-5, wherein the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8, 16, 24, 32, 53, 54, 55, 59, 62, 63, or 64.

Embodiment 7. The anti-ROR1 antibody or the antigen-binding fragment thereof according to any one of embodiments 1-6, wherein the anti-ROR1 antibody or the antigen-binding fragment thereof comprises:

(1) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;

(2) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;

(3) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24;

(4) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32;

(5) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53;

(6) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54;

(7) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55;

(8) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53;

(9) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54;

(10) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55;

(11) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 56, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(12) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 57, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(13) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 58, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(14) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62;

(15) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63;

(16) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64;

(17) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62;

(18) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63; or

(19) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64.

Embodiment 8. The anti-ROR1 antibody or the antigen-binding fragment thereof according to any one of embodiments 1-7, wherein the anti-ROR1 antibody or the antigen-binding fragment thereof comprises:

(1) a heavy chain variable region comprising the amino acid sequence set forth in SEQ ID NO: 7 and a light chain variable region comprising the amino acid sequence set forth in SEQ ID NO: 8;
(2) a heavy chain variable region comprising the amino acid sequence set forth in SEQ ID NO: 15 and a light chain variable region comprising the amino acid sequence set forth in SEQ ID NO: 16;
(3) a heavy chain variable region comprising the amino acid sequence set forth in SEQ ID NO: 23 and a light chain variable region comprising the amino acid sequence set forth in SEQ ID NO: 24;
(4) a heavy chain variable region comprising the amino acid sequence set forth in SEQ ID NO: 31 and a light chain variable region comprising the amino acid sequence set forth in SEQ ID NO: 32;
(5) a heavy chain variable region comprising the amino acid sequence set forth in SEQ ID NO: 51 and a light chain variable region comprising the amino acid sequence set forth in SEQ ID NO: 53;
(6) a heavy chain variable region comprising the amino acid sequence set forth in SEQ ID NO: 51 and a light chain variable region comprising the amino acid sequence set forth in SEQ ID NO: 54;
(7) a heavy chain variable region comprising the amino acid sequence set forth in SEQ ID NO: 51 and a light chain variable region comprising the amino acid sequence set forth in SEQ ID NO: 55;
(8) a heavy chain variable region comprising the amino acid sequence set forth in SEQ ID NO: 52 and a light chain variable region comprising the amino acid sequence set forth in SEQ ID NO: 53;
(9) a heavy chain variable region comprising the amino acid sequence set forth in SEQ ID NO: 52 and a light chain variable region comprising the amino acid sequence set forth in SEQ ID NO: 54;
(10) a heavy chain variable region comprising the amino acid sequence set forth in SEQ ID NO: 52 and a light chain variable region comprising the amino acid sequence set forth in SEQ ID NO: 55;
(11) a heavy chain variable region comprising the amino acid sequence set forth in SEQ ID NO: 56 and a light chain variable region comprising the amino acid sequence set forth in SEQ ID NO: 59;
(12) a heavy chain variable region comprising the amino acid sequence set forth in SEQ ID NO: 57 and a light chain variable region comprising the amino acid sequence set forth in SEQ ID NO: 59;
(13) a heavy chain variable region comprising the amino acid sequence set forth in SEQ ID NO: 58 and a light chain variable region comprising the amino acid sequence set forth in SEQ ID NO: 59;
(14) a heavy chain variable region comprising the amino acid sequence set forth in SEQ ID NO: 60 and a light chain variable region comprising the amino acid sequence set forth in SEQ ID NO: 62;
(15) a heavy chain variable region comprising the amino acid sequence set forth in SEQ ID NO: 60 and a light chain variable region comprising the amino acid sequence set forth in SEQ ID NO: 63;
(16) a heavy chain variable region comprising the amino acid sequence set forth in SEQ ID NO: 60 and a light chain variable region comprising the amino acid sequence set forth in SEQ ID NO: 64;
(17) a heavy chain variable region comprising the amino acid sequence set forth in SEQ ID NO: 61 and a light chain variable region comprising the amino acid sequence set forth in SEQ ID NO: 62;
(18) a heavy chain variable region comprising the amino acid sequence set forth in SEQ ID NO: 61 and a light chain variable region comprising the amino acid sequence set forth in SEQ ID NO: 63; or
(19) a heavy chain variable region comprising the amino acid sequence set forth in SEQ ID NO: 61 and a light chain variable region comprising the amino acid sequence set forth in SEQ ID NO: 64.

Embodiment 9. The anti-ROR1 antibody or the antigen-binding fragment thereof according to any one of embodiments 1-8, wherein the antigen-binding fragment is a Fab fragment, a F(ab')2 fragment, an Fd fragment, an Fv fragment, an isolated CDR region, or an scFv.

Embodiment 10. The anti-ROR1 antibody or the antigen-binding fragment thereof according to any one of embodiments 1-9, wherein the anti-ROR1 antibody or the antigen-binding fragment thereof is murine, chimeric, or humanized.

Embodiment 11. The anti-ROR1 antibody or the antigen-binding fragment thereof according to any one of embodiments 1-10, wherein the anti-ROR1 antibody is of the IgG1, IgG2, IgG3, or IgG4 isotype.

Embodiment 12. The anti-ROR1 antibody or the antigen-binding fragment thereof according to any one of embodiments 1-11, wherein the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33, 37, 41, 45, 65, 67, 75, 77, 79, 83, or 85.

Embodiment 13. The anti-ROR1 antibody or the antigen-binding fragment thereof according to any one of embodiments 1-12, wherein the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35, 39, 43, 47, 69, 71, 73, 81, 87, 89, or 91.

Embodiment 14. The anti-ROR1 antibody or the antigen-binding fragment thereof according to any one of embodiments 1-13, wherein the anti-ROR1 antibody comprises:

(1) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35;

(2) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39;

(3) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 43;

(4) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 45, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 47;

(5) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

(6) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71;

(7) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73;

(8) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

(9) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71;

(10) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73;

(11) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(12) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(13) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(14) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(15) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89;

(16) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91;

(17) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;

(18) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89; or

(19) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91.

Embodiment 15. The anti-ROR1 antibody or the antigen-binding fragment thereof according to any one of embodiments 1-14, wherein the anti-ROR1 antibody comprises:

(1) a heavy chain comprising the amino acid sequence set forth in SEQ ID NO: 33 and a light chain comprising the amino acid sequence set forth in SEQ ID NO: 35;

(2) a heavy chain comprising the amino acid sequence set forth in SEQ ID NO: 37 and a light chain comprising the amino acid sequence set forth in SEQ ID NO: 39;

(3) a heavy chain comprising the amino acid sequence set forth in SEQ ID NO: 41 and a light chain comprising the amino acid sequence set forth in SEQ ID NO: 43;

(4) a heavy chain comprising the amino acid sequence set forth in SEQ ID NO: 45 and a light chain comprising the amino acid sequence set forth in SEQ ID NO: 47;

(5) a heavy chain comprising the amino acid sequence set forth in SEQ ID NO: 65 and a light chain comprising the amino acid sequence set forth in SEQ ID NO: 69;

(6) a heavy chain comprising the amino acid sequence set forth in SEQ ID NO: 65 and a light chain comprising the amino acid sequence set forth in SEQ ID NO: 71;

(7) a heavy chain comprising the amino acid sequence set forth in SEQ ID NO: 65 and a light chain comprising the amino acid sequence set forth in SEQ ID NO: 73;

(8) a heavy chain comprising the amino acid sequence set forth in SEQ ID NO: 67 and a light chain comprising the amino acid sequence set forth in SEQ ID NO: 69;

(9) a heavy chain comprising the amino acid sequence set forth in SEQ ID NO: 67 and a light chain comprising the amino acid sequence set forth in SEQ ID NO: 71;

(10) a heavy chain comprising the amino acid sequence set forth in SEQ ID NO: 67 and a light chain comprising the amino acid sequence set forth in SEQ ID NO: 73;

(11) a heavy chain comprising the amino acid sequence set forth in SEQ ID NO: 75 and a light chain comprising the amino acid sequence set forth in SEQ ID NO: 81;

(12) a heavy chain comprising the amino acid sequence set forth in SEQ ID NO: 77 and a light chain comprising the amino acid sequence set forth in SEQ ID NO: 81;

(13) a heavy chain comprising the amino acid sequence set forth in SEQ ID NO: 79 and a light chain comprising the amino acid sequence set forth in SEQ ID NO: 81;

(14) a heavy chain comprising the amino acid sequence set forth in SEQ ID NO: 83 and a light chain comprising the amino acid sequence set forth in SEQ ID NO: 87;

(15) a heavy chain comprising the amino acid sequence set forth in SEQ ID NO: 83 and a light chain comprising the amino acid sequence set forth in SEQ ID NO: 89;

(16) a heavy chain comprising the amino acid sequence set forth in SEQ ID NO: 83 and a light chain comprising the amino acid sequence set forth in SEQ ID NO: 91;

(17) a heavy chain comprising the amino acid sequence set forth in SEQ ID NO: 85 and a light chain comprising the amino acid sequence set forth in SEQ ID NO: 87;

(18) a heavy chain comprising the amino acid sequence set forth in SEQ ID NO: 85 and a light chain comprising the amino acid sequence set forth in SEQ ID NO: 89; or

(19) a heavy chain comprising the amino acid sequence set forth in SEQ ID NO: 85 and a light chain comprising the amino acid sequence set forth in SEQ ID NO: 91.

Embodiment 16. The anti-ROR1 antibody or the antigen-binding fragment thereof according to any one of embodiments 1-15, wherein the anti-ROR1 antibody or the antigen-binding fragment thereof has one or several of the following properties and characteristics:

(1) binding to human ROR1; preferably, binding to human ROR1 with $K_D$ of 5E-08 M or less, 1E-09 M or less, or 5E-10 M or less, as measured by surface plasmon resonance;

(2) binding to monkey ROR1; preferably, binding to human ROR1 with $K_D$ of 5E-08 M or less, 1E-09 M or less, or 5E-10 M or less, as measured by surface plasmon resonance;

(3) inhibiting the Wnt5a-stimulated non-classical Wnt signaling pathway, wherein preferably, the anti-ROR1 antibody or the antigen-binding fragment thereof has an inhibition rate of 10% or higher, 15% or higher, or 20% or higher against the Wnt5a-stimulated non-classical Wnt signaling pathway at a concentration of 20 nM;

(4) exerting an internalization effect in cells expressing ROR1;

(5) exerting an ADCC effect on cells expressing ROR1;

(6) inducing the regression of tumor growth *in vivo*; and

(7) delaying tumor growth *in vivo*.

Embodiment 17. The anti-ROR1 antibody or the antigen-binding fragment thereof according to any one of embodiments 1-16, wherein the anti-ROR1 antibody or the antigen-binding fragment thereof is a monospecific antibody or a multispecific antibody.

Embodiment 18. An isolated nucleic acid, comprising a nucleotide sequence encoding the anti-ROR1 antibody or the antigen-binding fragment thereof according to any one of embodiments 1-17.

Embodiment 19. A vector, comprising the nucleic acid according to embodiment 18.

Embodiment 20. A host cell, comprising the nucleic acid according to embodiment 18 or the vector according to embodiment 19.

Embodiment 21. A method for preparing the anti-ROR1 antibody or the antigen-binding fragment thereof according to any one of embodiments 1-17, comprising culturing the host cell according to embodiment 20 to express the anti-ROR1 antibody or the antigen-binding fragment thereof, and isolating and purifying the anti-ROR1 antibody or the antigen-binding fragment thereof in the system.

Embodiment 22. A fusion protein, comprising the anti-ROR1 antibody or the antigen-binding fragment thereof according to any one of embodiments 1-17.

Embodiment 23. An antibody-drug conjugate, comprising the anti-ROR1 antibody or the antigen-binding fragment thereof according to any one of embodiments 1-17.

Embodiment 24. A chimeric antigen receptor, comprising the anti-ROR1 antibody or the antigen-binding fragment thereof according to any one of embodiments 1-17.

Embodiment 25. An engineered immune effector cell, comprising the chimeric antigen receptor according to embodiment 24.

Embodiment 26. A pharmaceutical composition, comprising the anti-ROR1 antibody or the antigen-binding fragment thereof according to any one of embodiments 1-17, the fusion protein according to embodiment 22, the antibody-drug conjugate according to embodiment 23, or the engineered immune effector cell according to embodiment 25, and one or more pharmaceutically acceptable excipients.

Embodiment 27. Use of the anti-ROR1 antibody or the antigen-binding fragment thereof according to any one of embodiments 1-17, the fusion protein according to embodiment 22, the antibody-drug conjugate according to embodiment 23, the engineered immune effector cell according to embodiment 25, or the pharmaceutical composition according to embodiment 26 for preparing a medicament for treating a disease expressing ROR1.

Embodiment 28. The use according to embodiment 27, wherein the disease is a tumor.

Embodiment 29. The use according to embodiment 28, wherein the tumor is leukemia, lymphoma, multiple myeloma, breast cancer, ovarian cancer, ovarian teratoma, colon cancer, lung cancer, skin cancer, pancreatic cancer, testicular cancer, bladder cancer, uterine cancer, prostate cancer, or adrenal cancer.

Embodiment 30. A method for treating a disease expressing ROR1, comprising administering to a subject in need a therapeutically effective amount of the anti-ROR1 antibody or the antigen-binding fragment thereof according to any one of embodiments 1-17, the fusion protein according to embodiment 22, the antibody-drug conjugate according to embodiment 23, the engineered immune effector cell according to embodiment 25, or the pharmaceutical composition according to embodiment 26.

Embodiment 31. The method according to embodiment 30, wherein the disease is a tumor.

Embodiment 32. The method according to embodiment 31, wherein the tumor is leukemia, lymphoma, multiple myeloma, breast cancer, ovarian cancer, ovarian teratoma, colon cancer, lung cancer, skin cancer, pancreatic cancer, testicular cancer, bladder cancer, uterine cancer, prostate cancer, or adrenal cancer.

Embodiment 33. A kit, comprising the anti-ROR1 antibody or the antigen-binding fragment thereof according to any one of embodiments 1-17, the fusion protein according to embodiment 22, the antibody-drug conjugate according to embodiment 23, the engineered immune effector cell according to embodiment 25, or the pharmaceutical composition according to embodiment 26.

For clarity, the present disclosure is further described with the following examples, which are, however, not intended to limit the scope of the present disclosure. Those skilled in the art will readily identify a variety of noncritical parameters that may be changed or modified to produce substantially similar results.

UC-961 used in the examples is Zilovertamab from Oncternal® Therapeutics. The isotype IgG used in Examples 8 and 10 is a human IgG1 antibody (Sino Biological®, Cat. No.: HG1K). The cell information involved in the examples of the present disclosure is shown in the table below:

| Cell | Cell source | Cancer type | ROR1 expression level |
|------|-------------|-------------|------------------------|
| A549 | Cell Resource Center, Institute of Basic Medical Sciences, Chinese Academy of Medical Sciences, Cat. No.: PUMC000002 | Human lung cancer cell | Medium |
| PA-1 | Shanghai Jinyuan Biotechnology, Cat. No.: SC323 | Human ovarian teratoma cell | High |
| Jeko-1 | Shanghai Institute of Biochemistry and Cell Biology, Chinese Academy of Sciences, Cat. No.: TCHu194 | Human mantle cell lymphoma cell | Medium |
| SW620 | Nanjing Cobioer Biosciences, Cat. No.: CBP60036 | Human colon cancer cell | Low |
| NCI-H1993 | Beijing BeNa Culture Collection, Cat. No.: BNCC342186 | Human lung cancer cell | Medium |
| MDA-MB-468 | Beijing BeNa Culture Collection, Cat. No.: BNCC339862 | Human breast cancer cell | Medium |
| NCI-1975 | Shanghai Institute of Biochemistry and Cell Biology, Chinese Academy of Sciences, Cat. No.: TCHu193 | Human lung cancer cell | Medium |

### Example 1: Preparation of ROR1 Antigen

A nucleotide sequence encoding a human ROR1 extracellular region-human Fc fusion protein (human ROR1 ECD-hFc) or a human/cynomolgus monkey ROR1 ECD-His tag recombinant protein (human/cyno ROR1 ECD-His) was inserted into an expression vector pcDNA3.1(+) (Invitrogen®) at an appropriate enzyme cutting site, and expression was performed in Expi293 cells (THERMO FISHER SCIENTIFIC®, Cat. No.: 100044202) to obtain human ROR1 ECD-hFc and human/cyno ROR1 ECD-His. The amino acid sequence of human ROR1 ECD-hFc is set forth in SEQ ID NO: 93; the amino acid sequence of human/cyno ROR1 ECD-His is set forth in SEQ ID NO: 94.

### Example 2: Generation of Anti-ROR1 Monoclonal Antibodies

Human ROR1 ECD-hFc was mixed and emulsified with Freund's complete adjuvant or Freund's incomplete adjuvant at a volume ratio of 1:1, and A/J mice, BALB/c mice, or SJL mice were immunized by subcutaneous injection at 25 μg/mouse every 2-3 weeks for 7-9 weeks. Immunizations starting from the second dose were boost immunizations. One week after each boost immunization, the serum of mice was collected, and the antibody titer in the serum was determined by ELISA. Mice with post-immunization serum antibody titers higher than $1:10^5$ were selected for cell fusion. 3 days before cell fusion, mice were subjected to final boost by intraperitoneal injection of human ROR1 ECD-hFc without adjuvant. Spleen cells from the immunized mice were fused with SP2/0 myeloma cells using the electrofusion system (BTX®, model: ECM®-2001). After fusion, the cells were resuspended in an SFM medium (Gibco®, Cat. No.: 11995-040) containing HAT (Gibco®, Cat. No.: 21060-017) and serum (Gibco®, Cat. No.: 10099-141C), added to a 96-well plate at 100 $\mu$L/well, and incubated in an incubator at 37 °C with 5% $CO_2$ for 7 days. The hybridoma cell culture supernatants were then collected for screening of anti-ROR1 monoclonal antibodies.

### Example 3: Screening of Anti-ROR1 Monoclonal Antibodies

The hybridoma cell culture supernatants were screened by ELISA using human/cyno ROR1 ECD-His to obtain positive clones. The positive clones were subcloned to obtain positive monoclones. The obtained positive monoclones were evaluated by flow cytometry (FACS) using the cell line Jeko-1, which naturally expresses ROR1. The positive monoclones m3G9B1, m29H5D8, m135A2B10, and m137G11D10 were obtained by screening.

### Example 4: Preparation of Chimeric Anti-ROR1 Antibodies

(1) Acquisition of cDNA

The hybridoma cells m3G9B1, m29H5D8, m135A2B10, and m137G11D10 were selected. Total RNA was isolated from the cells using a total RNA extraction kit (Takara®, Cat. No.: 9767) and used as a template. First-strand cDNA was synthesized using superscript III reverse transcriptase according to the kit instructions (THERMO FISHER SCIENTIFIC®, Cat. No.: 18080051). Using the first-strand cDNA as a template, the sequences of the variable regions of the mouse antibodies were amplified by PCR using a mouse IgG primer and a Kappa primer. The PCR mixture was electrophoretically separated in a 1% agarose/Tris-borate gel containing 0.5 $\mu$g/mL of ethidium bromide. A DNA fragment of the expected size was cut off from the gel and purified. The purified PCR product was cloned into the pMD-19T vector (Takara®, Cat. No.: 6013), which was then transfected into DH5$\alpha$ competent *E. coli* cells (Takara®, Cat. No.: 9057) for culturing. Single colonies were picked from the LB solid culture plate and subjected to DNA sequencing to obtain the sequences of the heavy chain variable regions and the light chain variable regions of the anti-ROR1 mouse antibodies, as shown in Table 1.

(2) Construction and expression of chimeric antibodies

The mouse VL region gene fragment was linked to a human $\kappa$ chain constant region gene fragment to construct a chimeric light chain expression plasmid, and the mouse VH region gene fragment was linked to a human IgG1 constant region gene fragment to construct a chimeric heavy chain expression plasmid. When the density of ExpiCHO-S cells reached $6 \times 10^6$ cells/mL, the chimeric heavy chain expression plasmid and the chimeric light chain expression plasmid corresponding to each antibody were co-transfected into ExpiCHO-S cells (THERMO FISHER SCIENTIFIC®, Cat. No.: A29127) at a ratio of 1:1 for protein expression. The transfected cells were cultured using ExpiCHO Expression Medium (THERMO FISHER SCIENTIFIC®, Cat. No.: A29100-01) at 37 °C with 8% $CO_2$. After 7-10 days of culturing, the chimeric antibodies in the cell culture supernatant were purified using a Protein A column (GE healthcare®, Cat. No.: 17-5474-02).

Table 1. Sequence information of anti-ROR1 mouse antibodies and chimeric antibodies (SEQ ID NO:)

| Mouse antibody/chimeric antibody | VH amino acid sequence | VL amino acid sequence | Heavy chain amino acid sequence/nucleotide sequence | Light chain amino acid sequence/nucleotide sequence |
|---|---|---|---|---|
| m3G9B1/ch3G9B1 | 7 | 8 | 33/34 | 35/36 |
| m29H5D8/ch29H5D8 | 15 | 16 | 37/38 | 39/40 |
| ml135A2B10/ch135A2B10 | 23 | 24 | 41/42 | 43/44 |
| m137G11D10/ch137G11D10 | 31 | 32 | 45/46 | 47/48 |
| Note: The mouse antibodies and the chimeric antibodies share the same VH and VL amino acid sequences. The heavy chain and light chain amino acid sequences and nucleotide sequences refer to the heavy chain and light chain amino acid sequences and nucleotide sequences of the chimeric anti-ROR1 antibodies. | | | | |

### Example 5: Assay on *In Vitro* Binding Activity of Chimeric Anti-ROR1 Antibodies

(1) Assay on binding of chimeric anti-ROR1 antibodies to ROR1 by surface plasmon resonance (SPR)

A certain amount of anti-ROR1 antibody was captured by an Anti-hIgG chip, and human/cynomolgus monkey ROR1 protein (Acro Biosystems®, Cat. No.: RO1-H522y) was then flowed over the chip surface. The reaction signal was detected in real-time using Biacore® to obtain association and dissociation curves. The buffer used in the experiment was a Biacore® universal buffer (137 mM NaCl, 2.7 mM KCl, 10 mM $Na_2HPO_4 \cdot 12H_2O$, 1.8 mM $KH_2PO_4$, 0.05% surfactant P-20, pH 7.4). Anti-hIgG was coupled to the surface of a CM5 chip to capture the chimeric anti-ROR1 antibodies and the reference standard (benchmark) UC-961 (MCE®, Cat. No.: HY-P99201). The signal values for the interaction between ROR1 protein at different concentrations (100 nM, 50 nM, 25 nM, 12.5 nM, 6.25 nM, and 3.125 nM) and the anti-ROR1 antibodies were then separately measured. The flow rate in the flow cell was 50 $\mu$L/min, the association time was 240 s, the dissociation time was 1400 s, and regeneration was performed with 3 M $MgCl_2$ (GE) for 60 s until the baseline was stable. Results were obtained by calculation according to the affinity and kinetics 1:1 binding mode in the Biacore® evaluation software. The affinity results are shown in Table 2.

Table 2. Binding affinity of chimeric anti-ROR1 antibodies for ROR1

| Antibody | $k_a$ (1/Ms) | $k_d$ (1/s) | $K_D$ (M) |
|---|---|---|---|
| ch3G9B1 | 2.36E+06 | 6.39E-02 | 2.71E-08 |
| ch29H5D8 | 3.70E+06 | 2.48E-03 | 6.69E-10 |
| ch135A2B10 | 9.41E+05 | 5.51E-04 | 5.86E-10 |
| ch137G11D10 | 5.16E+06 | 3.15E-03 | 6.10E-10 |
| UC-961 | 1.33E+06 | 2.73E-02 | 2.06E-08 |

(2) Assay on binding of chimeric anti-ROR1 antibodies to cells by FACS

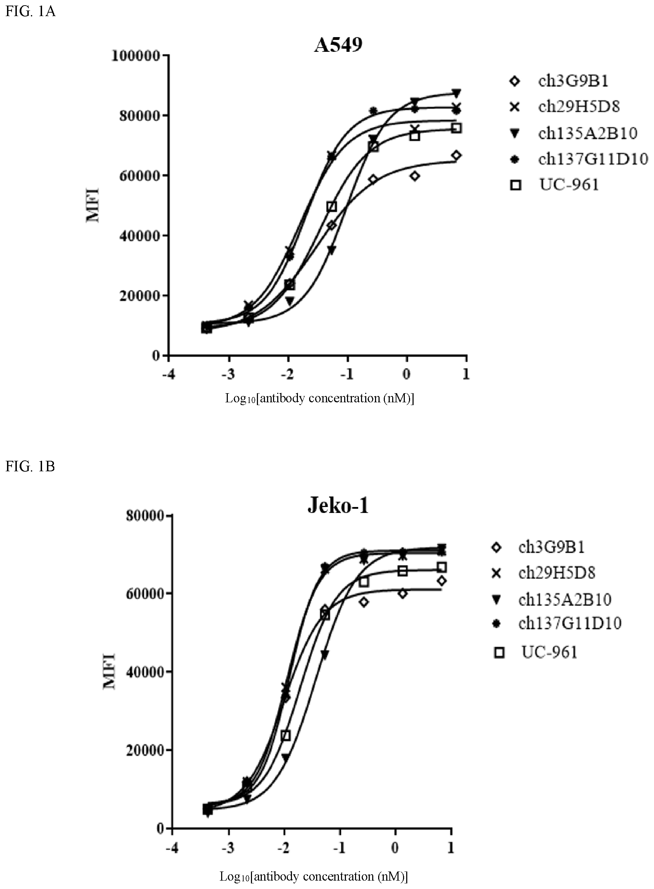

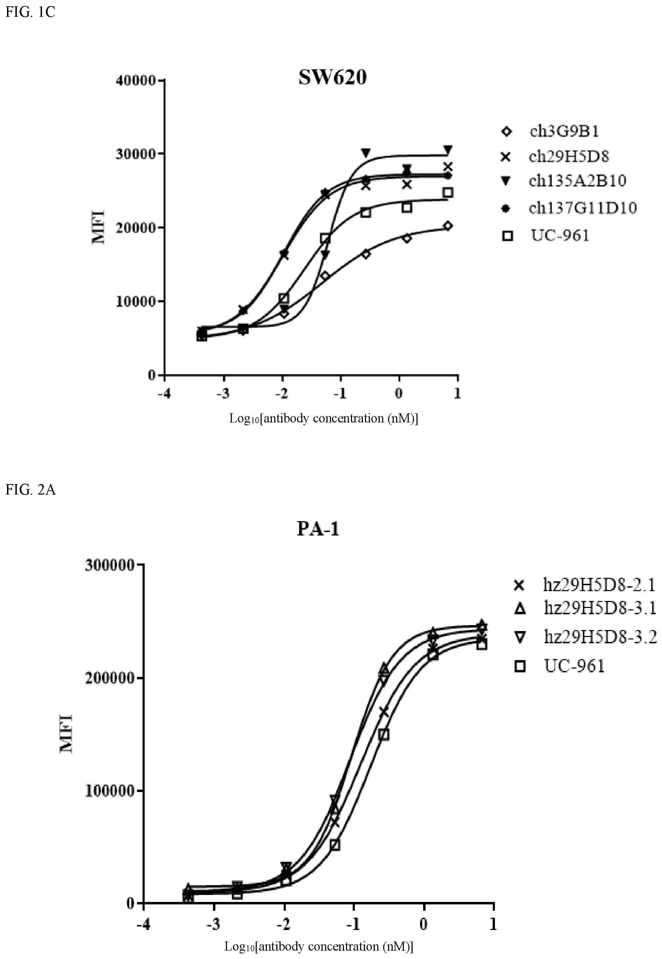

The cell lines A549, SW620, and Jeko-1, which naturally express ROR1, were added to a 96-well plate at $1 \times 10^5$ cells/well. Gradient-diluted anti-ROR1 antibodies (starting dilution concentration of 6.67 nM, 5-fold dilution, 7 concentrations) were added, and the plate was incubated at 4 °C for 0.5 h. After the 96-well plate was washed three times with an FACS buffer (Miltenyi®, Cat. No.: 130-091-221), a secondary antibody PE anti-human IgG Fc (Jackson®, Cat. No.: 109-116-170) was diluted at a ratio of 1:200 and added to the 96-well plate at 100 $\mu$L/well, and the plate was incubated at 4 °C for 20 min. The cells were washed three times with an FACS buffer and detected and analyzed using a flow cytometer (Sartorius®, model: iQue3). The data were analyzed, and $EC_{50}$ was calculated. The results are shown in Table 3 and FIGs. 1A-1C.

Table 3. Binding $EC_{50}$ and maximum binding values of chimeric anti-ROR1 antibodies to cells expressing ROR1

| Antibody | A549 | | Jeko-1 | | SW620 | |
|---|---|---|---|---|---|---|
| | $EC_{50}$ (nM) | Top (MFI) | $EC_{50}$ (nM) | Top (MFI) | $EC_{50}$ (nM) | Top (MFI) |
| ch3G9B1 | 0.030 | 65190 | 0.010 | 61101 | 0.048 | 20351 |
| ch29H5D8 | 0.016 | 78507 | 0.011 | 70403 | 0.010 | 26995 |
| ch135A2B10 | 0.094 | 87778 | 0.037 | 71864 | 0.060 | 29797 |
| ch137G11D10 | 0.020 | 82848 | 0.012 | 71054 | 0.010 | 27276 |
| UC-961 | 0.035 | 75684 | 0.019 | 66065 | 0.024 | 23851 |

**Example 6: Humanization of Anti-ROR1 Antibodies**

Three antibodies with better binding activity, m29H5D8, m135A2B10, and m137G11D10, were preferably selected for humanization. The m29H5D8, m135A2B10, and m137G11D10 antibodies were humanized using the CDR grafting method. Human germline antibody sequences with high homology to the mouse m29H5D8, m135A2B10, and m137G11D10 antibodies were selected. The complementarity determining regions (CDRs) from the VH and VL of the murine m29H5D8, m135A2B10, and m137G11D10 antibodies were grafted onto the template human antibody. Several amino acid residue positions in the framework regions (FRs) of the template human antibody VH and VL were back-mutated to the corresponding amino acid residues in the mouse m29H5D8, m135A2B10, and m137G11D10 antibodies to obtain a humanized light chain variable region and a humanized heavy chain variable region.

The humanized light chain variable region described above was linked to the human κ chain constant region to construct a humanized light chain. The humanized heavy chain variable region described above was linked to the human IgG1 constant region to construct a humanized heavy chain. The humanized antibodies were constructed and expressed by referring to the method in Example 4. The sequence information of the humanized anti-ROR1 antibodies is shown in Table 4.

Table 4. Sequence information of humanized anti-ROR1 antibodies (SEQ ID NO:)

| Antibody | VH amino acid sequence | VL amino acid sequence | Heavy chain amino acid sequence/nucleotide sequence | Light chain amino acid sequence/nucleotide sequence |
|---|---|---|---|---|
| hz29H5D8-1.1 | 51 | 53 | 65/66 | 69/70 |
| hz29H5D8-1.2 | 52 | 53 | 67/68 | 69/70 |
| hz29H5D8-2.1 | 51 | 54 | 65/66 | 71/72 |
| hz29H5D8-2.2 | 52 | 54 | 67/68 | 71/72 |
| hz29H5D8-3.1 | 51 | 55 | 65/66 | 73/74 |
| hz29H5D8-3.2 | 52 | 55 | 67/68 | 73/74 |
| hz135A2B10-1.1 | 56 | 59 | 75/76 | 81/82 |
| hz135A2B10-1.2 | 57 | 59 | 77/78 | 81/82 |
| hz135A2B10-1.3 | 58 | 59 | 79/80 | 81/82 |
| hz137G11D10-1.1 | 60 | 62 | 83/84 | 87/88 |
| hz137G11D10-1.2 | 61 | 62 | 85/86 | 87/88 |
| hz137G11D10-2.1 | 60 | 63 | 83/84 | 89/90 |
| hz137G11D10-2.2 | 61 | 63 | 85/86 | 89/90 |
| hz137G11D10-3.1 | 60 | 64 | 83/84 | 91/92 |
| hz137G11D10-3.2 | 61 | 64 | 85/86 | 91/92 |

## Example 7: Assay on Binding Activity of Humanized Anti-ROR1 Antibodies

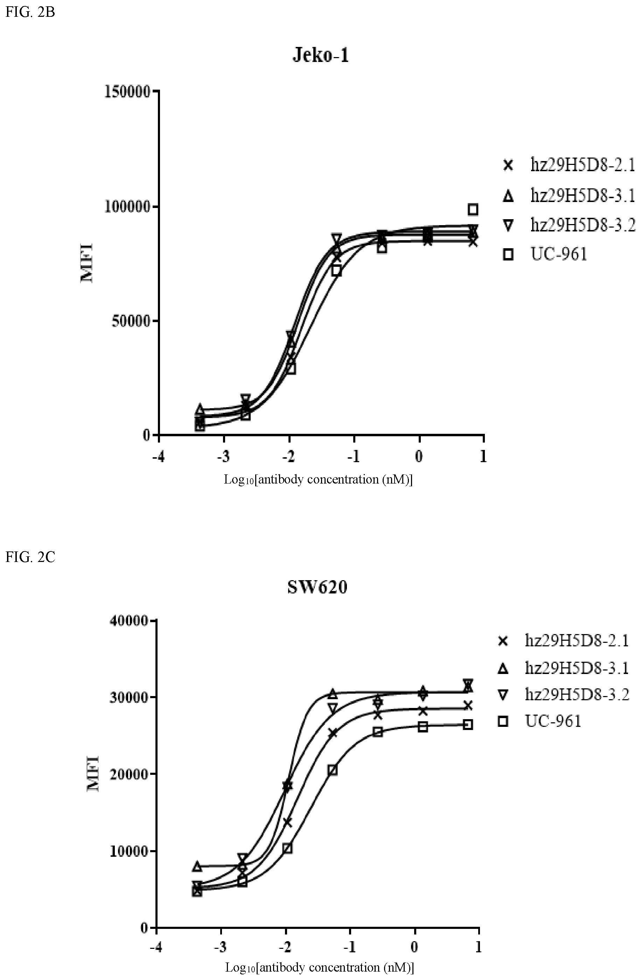

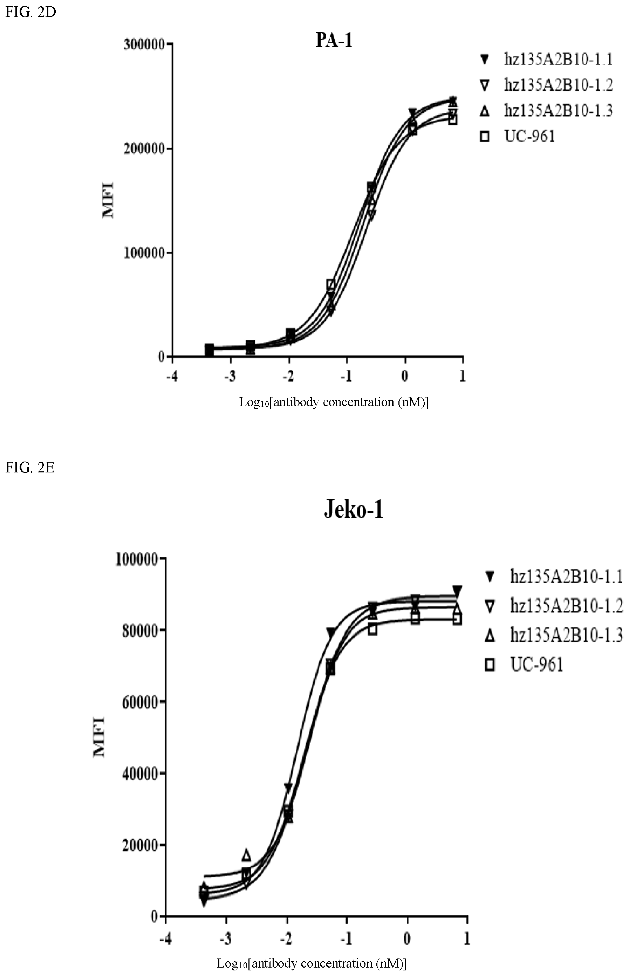

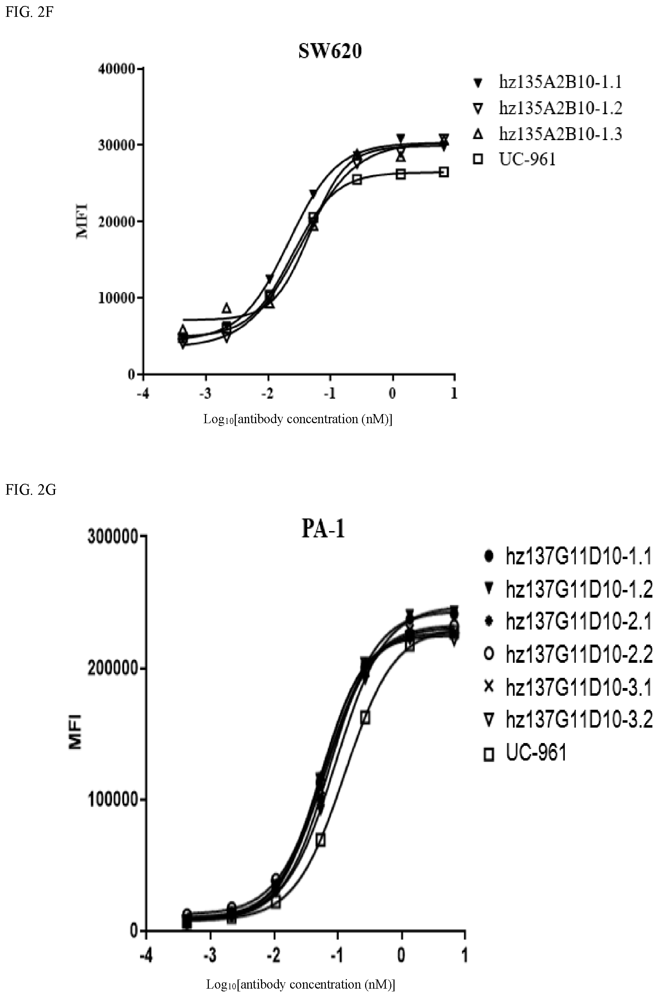

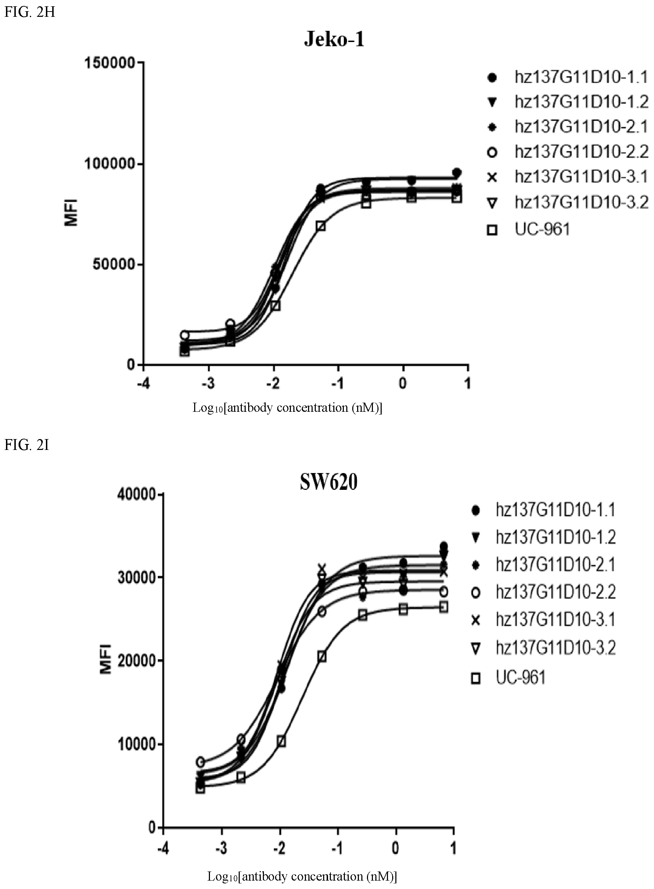

The binding of the humanized anti-ROR1 antibodies to ROR1 was assayed by SPR, and the binding of the humanized anti-ROR1 antibodies to cells naturally expressing ROR1 was assayed by FACS. Reference was made to Example 5 for the experimental method, wherein the cells used in the assay on the binding of the humanized anti-ROR1 antibodies to cells naturally expressing ROR1 were PA-1, Jeko-1, and SW620. The binding affinity results of the humanized anti-ROR1 antibodies to ROR1 are shown in Table 5, and the binding results of the humanized anti-ROR1 antibodies to cells expressing ROR1 are shown in Tables 6-1 to 6-3 and FIGs. 2A-2I.

Table 5. Binding affinity of humanized anti-ROR1 antibodies for ROR1

| Antibody | (1/Ms) | $k_d$ (1/s) | $K_D$ (M) |
|---|---|---|---|
| hz29H5D8-2.1 | 1.57E+06 | 9.25E-04 | 5.89E-10 |
| hz29H5D8-3.1 | 1.95E+06 | 2.18E-04 | 1.12E-10 |
| hz29H5D8-3.2 | 1.79E+06 | 3.54E-04 | 1.97E-10 |
| hz135A2B10-1.1 | 9.68E+05 | 5.10E-04 | 5.27E-10 |
| hz135A2B10-1.2 | 1.05E+06 | 5.96E-04 | 5.69E-10 |
| hz135A2B10-1.3 | 9.85E+05 | 5.67E-04 | 5.75E-10 |
| hz137G11D10-1.1 | 3.72E+06 | 5.39E-04 | 1.45E-10 |
| hz137G11D10-1.2 | 3.94E+06 | 4.14E-04 | 1.05E-10 |
| hz137G11D10-2.1 | 2.74E+06 | 7.92E-04 | 2.89E-10 |

(continued)

| Antibody | (1/Ms) | $k_d$ (1/s) | $K_D$ (M) |
|---|---|---|---|
| hz137G11D10-2.2 | 2.37E+06 | 8.11E-04 | 3.43E-10 |
| hz137G11D10-3.1 | 2.86E+06 | 4.92E-04 | 1.72E-10 |
| hz137G11D10-3.2 | 2.92E+06 | 4.69E-04 | 1.60E-10 |
| UC-961 | 1.54E+06 | 1.80E-03 | 1.17E-09 |

Table 6-1. Binding $EC_{50}$ and maximum binding values of humanized anti-ROR1 antibodies to cells expressing ROR1

| Antibody | PA-1 | | Jeko-1 | | SW620 | |
|---|---|---|---|---|---|---|
| | $EC_{50}$ (nM) | Top (MFI) | $EC_{50}$ (nM) | Top (MFI) | $EC_{50}$ (nM) | Top (MFI) |
| hz29H5D8-2.1 | 0.126 | 238989 | 0.015 | 84983 | 0.015 | 28571 |
| hz29H5D8-3.1 | 0.092 | 246565 | 0.014 | 87737 | 0.011 | 30672 |
| hz29H5D8-3.2 | 0.087 | 243673 | 0.012 | 89015 | 0.010 | 30689 |
| UC-961 | 0.173 | 235461 | 0.021 | 91690 | 0.025 | 26461 |

Table 6-2. Binding $EC_{50}$ and maximum binding values of humanized anti-ROR1 antibodies to cells expressing ROR1

| Antibody | PA-1 | | Jeko-1 | | SW620 | |
|---|---|---|---|---|---|---|
| | $EC_{50}$ (nM) | Top (MFI) | $EC_{50}$ (nM) | Top (MFI) | $EC_{50}$ (nM) | Top (MFI) |
| hz135A2B10-1.1 | 0.166 | 249756 | 0.015 | 88344 | 0.022 | 30313 |
| hz135A2B10-1.2 | 0.217 | 238449 | 0.022 | 89733 | 0.034 | 30423 |
| hz135A2B10-1.3 | 0.192 | 249187 | 0.024 | 86676 | 0.047 | 29895 |
| UC-961 | 0.127 | 231668 | 0.019 | 83187 | 0.025 | 26461 |

Table 6-3. Binding $EC_{50}$ and maximum binding values of humanized anti-ROR1 antibodies to cells expressing ROR1

| Antibody | PA-1 | | Jeko-1 | | SW620 | |
|---|---|---|---|---|---|---|
| | $EC_{50}$ (nM) | Top (MFI) | $EC_{50}$ (nM) | Top (MFI) | $EC_{50}$ (nM) | Top (MFI) |
| hz137G11D10-1.1 | 0.075 | 243871 | 0.015 | 93169 | 0.013 | 32620 |
| hz137G11D10-1.2 | 0.092 | 247575 | 0.016 | 92532 | 0.013 | 31503 |
| hz137G11D10-2.1 | 0.053 | 227476 | 0.010 | 87963 | 0.009 | 29539 |
| hz137G11D10-2.2 | 0.061 | 233128 | 0.012 | 86077 | 0.009 | 28533 |
| hz137G11D10-3.1 | 0.063 | 231326 | 0.012 | 87039 | 0.009 | 30875 |
| hz137G11D10-3.2 | 0.054 | 225161 | 0.012 | 86753 | 0.011 | 30675 |
| UC-961 | 0.127 | 231668 | 0.019 | 83187 | 0.025 | 26461 |

**Example 8: Internalization Effects of Humanized Anti-ROR1 Antibodies**

The cDNA encoding human ROR1 (SEQ ID NO: 95) was obtained by gene synthesis and then subcloned into the expression vector pcDNA3.1(+). According to the instructions of the Lipofectamine 2000 transfection reagent (THERMO FISHER SCIENTIFIC®, Cat. No.: 11668019), the expression vector was transfected into NCI-1975, NCI-H1993, and MDA-MB-468 cells to obtain the stable cell lines NCI-1975-ROR1, NCI-H1993-ROR1, and MDA-MB-468-ROR1, respectively.

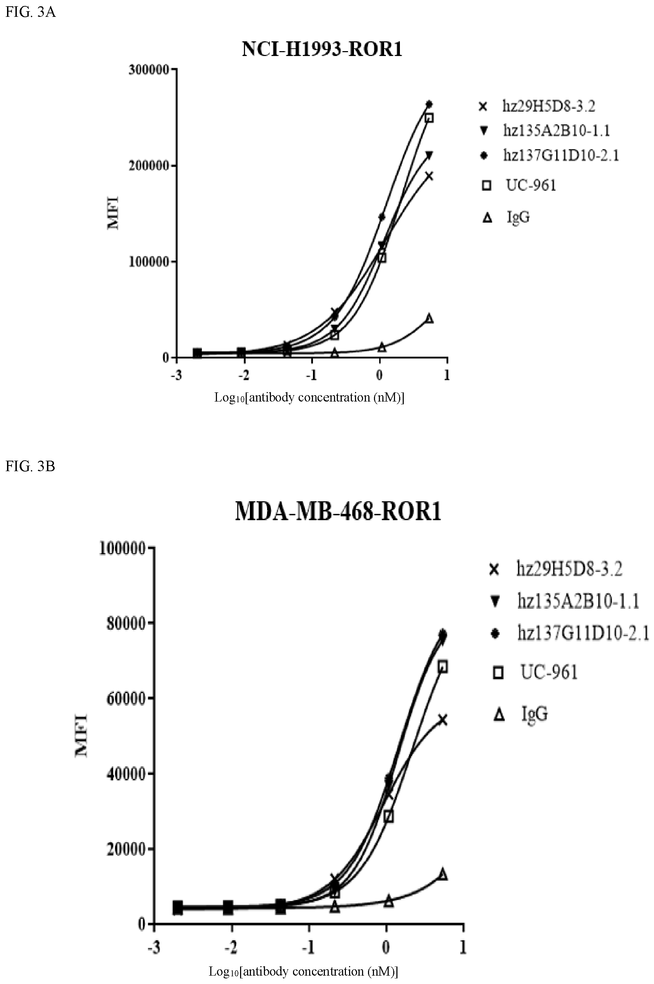

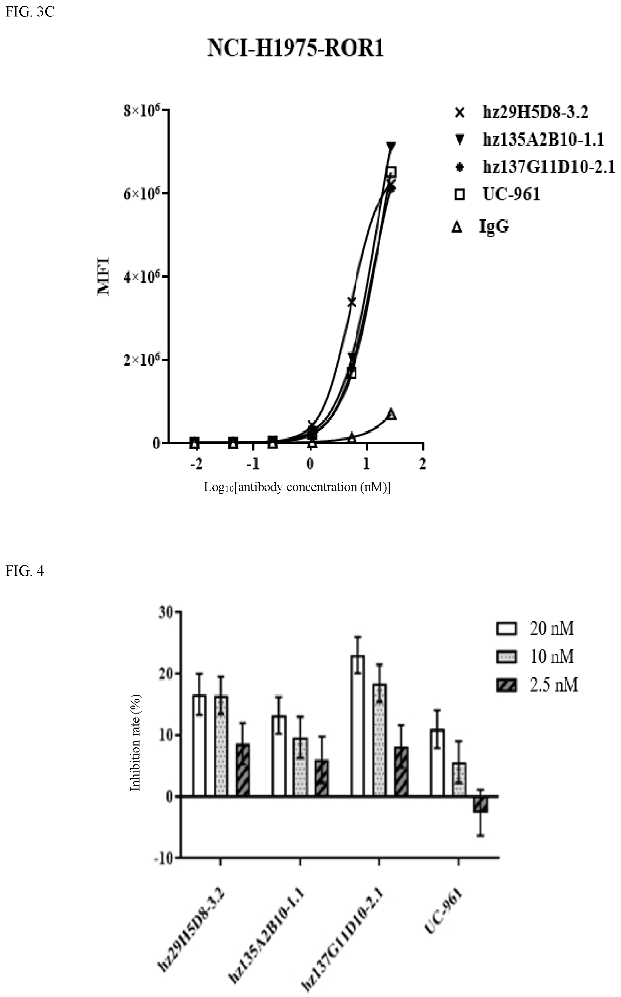

The internalization analysis of anti-ROR1 antibodies was performed based on their binding to the stably transfected cell strains NCI-1975-ROR1, NCI-H1993-ROR1, and MDA-MB-468-ROR1 stably expressing ROR1, using isotype IgG as a negative control. The cell density of the cells expressing ROR1 was adjusted to $2 \times 10^6$ cells/mL and added to a 96-well plate at 20 μL/well. The internalization detection reagent from the pH-dependent endocytosis detection kit (Sartorius®, Cat. No.: 90564) was mixed with the humanized anti-ROR1 antibody at a volume ratio of 1:1. For NCI-H1975-ROR1 cells, the final concentration of the humanized anti-ROR1 antibody was 26.7 nM; for NCI-H1993-ROR1 and MDA-MB-468-ROR1 cells, the final concentration of the humanized anti-ROR1 antibody was 5.3 nM. After incubation at 37 °C in the dark for 15 min, the mixture was diluted in a 5-fold gradient across 6 concentration gradients. This gradient-diluted co-incubation system was added to the 96-well plate at 20 μL/well and incubated at 37 °C for 2-3 h. Detection and analysis were performed using a flow cytometer (Sartorius, model: iQue3). The data were analyzed, and $EC_{50}$ was calculated. The results are shown in FIGs. 3A-3C, Table 7-1, and Table 7-2. These data indicate that hz29H5D8-3.2, hz135A2B10-1.1, and hz137G11D10-2.1 are capable of efficiently exerting internalization effects in cells expressing ROR1.

Table 7-1. Internalization effects of humanized anti-ROR1 antibodies in cells expressing ROR1

| | $EC_{50}$ (nM) | |
| --- | --- | --- |
| | NCI-H1993-ROR1 | MDA-MB-468-ROR1 |
| hz29H5D8-3.2 | 1.18 | 0.92 |
| hz135A2B10-1.1 | 1.16 | 1.39 |
| hz137G11D10-2.1 | 1.24 | 1.44 |
| UC-961 | 2.06 | 2.15 |

Table 7-2. Internalization effects of humanized anti-ROR1 antibodies in cells expressing ROR1

| | $EC_{50}$ (nM) |
| --- | --- |
| | NCI-H1975-ROR1 |
| hz29H5D8-3.2 | 5.19 |
| hz135A2B10-1.1 | 14.92 |
| hz137G11D10-2.1 | 12.42 |
| UC-961 | 17.68 |

**Example 9: Inhibitory Effects of Humanized Anti-ROR1 Antibodies on Wnt5a-Stimulated Non-Classical Wnt Signaling Pathway**

MEC-1 cells (Nanjing Cobioer Biosciences, Cat. No.: CBP60514) naturally express Wnt5a, a ligand of ROR1. Based on these cells, the inhibition of humanized anti-ROR1 antibodies against the Wnt5a-stimulated non-classical Wnt signaling pathway was analyzed.

The cDNA encoding human ROR1 (SEQ ID NO: 95) was obtained by gene synthesis and then subcloned into the expression vector pcDNA3.1(+). According to the instructions of the Lipofectamine 2000 transfection reagent (THERMO FISHER SCIENTIFIC®, Cat. No.: 11668019), the expression vector was transfected into 293T-NF-κB cells to obtain the stable cell line 293T-ROR1-NF-κB.

293T-ROR1-NF-κB cells stably expressing ROR1 were adjusted to a cell density of $2 \times 10^5$ cells/mL with a DMEM medium containing 10% FBS and seeded into a 96-well all-white plate (costar®, Cat. No.: 3917) at 100 μL/well, and the plate was incubated overnight. The supernatant was discarded from the 96-well all-white plate, and the humanized anti-ROR1 antibodies diluted with the MEC-1 cell supernatant (in-plate final concentrations of 20 nM, 10 nM, and 2.5 nM) were added at 100 μL/well. The plate was incubated in an incubator at 37 °C with 5% $CO_2$ for 24 h. A group without an antibody was set as a blank control group. The Bio-Lite Luciferase Assay System detection reagent (Vazyme®, Cat. No.: DD1201-03) was equilibrated to room temperature and added to the 96-well all-white plate at 100 μL/well. After 3 min of reaction, the chemiluminescence signal was detected using a multi-mode microplate reader (PE, model: Envision 2105). The data were analyzed, and the inhibition rate was calculated. The results are shown in FIG. 4. The results show that the humanized anti-ROR1 antibodies hz29H5D8-3.2, hz135A2B10-1.1, and hz137G11D10-2.1 all had inhibitory effects on the Wnt5a-stimulated non-classical Wnt signaling pathway, and all were superior to UC-961.

Inhibition rate (%) = [(RLU value of blank control group - RLU value of antibody group)/RLU value of blank control group] $\times$ 100%

**Example 10: ADCC Effects of Humanized Anti-ROR1 Antibodies**

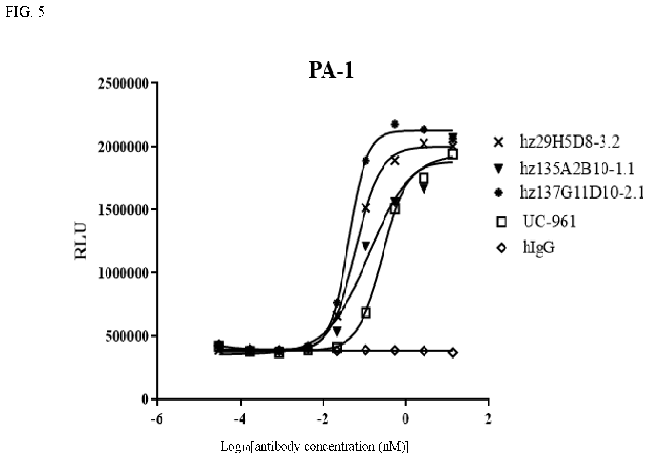

Based on the reporter gene method, PA-1 cells were used as target cells, recombinant Jurkat cells (BPS Bioscience™, Cat. No.: 60541) were used to detect the ADCC effects of humanized anti-ROR1 antibodies, and isotype IgG was used as a negative control. PA-1 cells were adjusted to a cell density of $2 \times 10^5$ cells/mL using an MEM medium containing 10% FBS, seeded into a 96-well all-white plate at 100 μL/well, and incubated overnight. The supernatant was discarded from the 96-well all-white plate, and the humanized anti-ROR1 antibodies diluted in a gradient (starting at an in-plate final concentration of 13.3 nM, 5-fold gradient dilution) with an experimental medium (RPMI-1640 medium containing 2% FBS) were added at 50 μL/well. The recombinant Jurkat cells were adjusted to a cell density of $4 \times 10^6$ cells/mL using an experimental medium, seeded into the 96-well all-white plate at 50 μL/well, and incubated in an incubator at 37 °C with 5% $CO_2$ for 6 h. The Bio-Lite Luciferase Assay System detection reagent (Vazyme®, Cat. No.: DD1201-03) was equilibrated to room temperature and added to the 96-well all-white plate at 100 μL/well. After 3 min of reaction, the chemiluminescence signal was detected using a multi-mode microplate reader. The data were analyzed, and $EC_{50}$ was calculated. The ADCC effects of the humanized anti-ROR1 antibodies are shown in FIG. 5, and the calculated $EC_{50}$ is shown in Table 8.

Table 8. ADCC effects of humanized anti-ROR1 antibodies on PA-1 cells

| Humanized antibody | $EC_{50}$ (nM) |
|---|---|
| hz29H5D8-3.2 | 0.06 |
| hz135A2B10-1.1 | 0.13 |
| hz137G11D10-2.1 | 0.04 |
| UC-961 | 0.27 |

The sequence information of the present disclosure is summarized in Table S2 below.

Table S2. Sequence information

| Description<br>Sequence (SEQ ID NO:) |
|---|
| HCDR1 of m3G9B1 and chimeric antibody thereof<br>SYGIT (SEQ ID NO: 1) |
| HCDR2 of m3G9B1 and chimeric antibody thereof<br>EIYPRSGNTYFNEKFKG (SEQ ID NO: 2) |
| HCDR3 of m3G9B1 and chimeric antibody thereof<br>DRTGTGVDDY (SEQ ID NO: 3) |
| LCDR1 of m3G9B1 and chimeric antibody thereof<br>ITNTDIDDDMS (SEQ ID NO: 4) |
| LCDR2 of m3G9B1 and chimeric antibody thereof<br>EGNTLRP (SEQ ID NO: 5) |
| LCDR3 of m3G9B1 and chimeric antibody thereof<br>LQSNNLPYT (SEQ ID NO: 6) |
| VH of m3G9B1 and chimeric antibody thereof<br><br>QVQLQQSGTELARPGASVKLSCKASGYTFTSYGITWVKQRTGQGLEWIGEIYPRSGNTYFNEKFKGKA<br>TLTADKSSSTAYMELRSLTSEDSAVYFCARDRTGTGVDDYWGQGTTLTVSS (SEQ ID NO: 7) |
| VL of m3G9B1 and chimeric antibody thereof<br><br>DIVLTQSPASLSMAIGEKVTIRCITNTDIDDDMSWYQQKPGEPPNLLISEGNTLRPGVPSRFSSSGYGRDF<br>VFTIENMLSEDVADYYCLQSNNLPYTFGGGTKLEIK (SEQ ID NO: 8) |
| HCDR1 of m29H5D8 and chimeric and humanized antibodies thereof<br>NYGIS (SEQ ID NO: 9) |
| HCDR2 of m29H5D8, ch29H5D8, hz29H5D8-1.2, hz29H5D8-2.2, and hz29H5D8-3.2 |

(continued)

| Description<br>Sequence (SEQ ID NO:) |
| --- |
| EIYPRSVNTYYSAKFKG (SEQ ID NO: 10) |
| HCDR3 of m29H5D8 and chimeric and humanized antibodies thereof<br>HYYGSSLDY (SEQ ID NO: 11) |
| LCDR1 of m29H5D8 and chimeric and humanized antibodies thereof<br>ITSSDIEDDMN (SEQ ID NO: 12) |
| LCDR2 of m29H5D8 and chimeric and humanized antibodies thereof<br>EGNSLRP (SEQ ID NO: 13) |
| LCDR3 of m29H5D8 and chimeric and humanized antibodies thereof<br>LQSDNIFT (SEQ ID NO: 14) |
| VH of m29H5D8 and chimeric antibody thereof<br>EVQLQQSGAELARPGASVKLSCKASGYTFTNYGISWVKQRTGQGLEWIGEIYPRSVNTYYSAKFKGK<br>ATLTADKSSSTAYMELRSLTSEDSAVYFCARHYYGSSLDYWGQGTTLTVSS (SEQ ID NO: 15) |
| VL of m29H5D8 and chimeric antibody thereof<br>DIVLTQSPASLSVATGEKVTIRCITSSDIEDDMNWYQQKPGEPPKLLISEGNSLRPGVPSRFSSSGYGTDF<br>VFTIENTLSEDVADYYCLQSDNIFTFGSGTKLEIK (SEQ ID NO: 16) |
| HCDR1 of m135A2B10 and chimeric and humanized antibodies thereof<br>TYGMA (SEQ ID NO: 17)<br>HCDR2 of m135A2B10 and chimeric and humanized antibodies thereof<br>SIHKGGFSTYYPDNVKG (SEQ ID NO: 18) |
| HCDR3 of m135A2B10 and chimeric and humanized antibodies thereof<br>HDWDGAMDY (SEQ ID NO: 19) |
| LCDR1 of m135A2B10 and chimeric and humanized antibodies thereof<br>KASQDINSYLN (SEQ ID NO: 20) |
| LCDR2 of m135A2B10 and chimeric and humanized antibodies thereof<br>RANRLVD (SEQ ID NO: 21) |
| LCDR3 of m135A2B10 and chimeric and humanized antibodies thereof<br>LQFDEFPYT (SEQ ID NO: 22) |
| VH of m135A2B10 and chimeric antibody thereof<br>DVKLVESGGGLVTPEGSLKLSCAASGFAFSTYGMAWVRQTPEKRLEWVASIHKGGFSTYYPDNVKGR<br>FTISRDNDKNTLYLQMSSLKSEDTAMYYCIRHDWDGAMDYWGQGTSVTVSS (SEQ ID NO: 23) |
| VL of m135A2B10 and chimeric antibody thereof<br>DIVLTQSPSSMYASLGERVTITCKASQDINSYLNWFQQKPGKSPKTLIYRANRLVDGVPSRFSGSGSGQD<br>YSLTISSLEYEDLGIYYCLQFDEFPYTFGGGTKLEIK (SEQ ID NO: 24) |
| HCDR1 of m137G11D10 and chimeric and humanized antibodies thereof<br>SDYAWN (SEQ ID NO: 25) |
| HCDR2 of m137G11D10 and chimeric and humanized antibodies thereof<br>YITYSASTSYNPSLKS (SEQ ID NO: 26) |
| HCDR3 of m137G11D10 and chimeric and humanized antibodies thereof<br>GAGTYYFDY (SEQ ID NO: 27) |
| LCDR1 of m137G11D10 and chimeric and humanized antibodies thereof<br>ITSTDIDEDMN (SEQ ID NO: 28) |
| LCDR2 of m137G11D10 and chimeric and humanized antibodies thereof<br>EDNSLRP (SEQ ID NO: 29) |

(continued)

| Description<br>Sequence (SEQ ID NO:) |
|---|
| LCDR3 of m137G11D10 and chimeric and humanized antibodies thereof<br>LQTNNLPLT (SEQ ID NO: 30) |
| VH of m137G11D10 and chimeric antibody thereof<br><br>DVQLQESGPGLVKPSQSLSLTCTVTGYSITSDYAWNWIRQFPGNKLEWMGYITYSASTSYNPSLKSRISI TRDTSKNQFFLQLNSVTTEDTATYYCARGAGTYYFDYWGQGSTLTVSS (SEQ ID NO: 31) |
| VL of m137G11D10 and chimeric antibody thereof<br>DIVLTQSPASLSVATGEKVTIRCITSTDIDEDMNWYQQKPGEPPKLLISEDNSLRPGVPSRFSSSGYGTDF VLTIENTLSEDVADYYCLQTNNLPLTFGAGTKLEIK (SEQ ID NO: 32) |
| Heavy chain of ch3G9B1<br><br>QVQLQQSGTELARPGASVKLSCKASGYTFTSYGITWVKQRTGQGLEWIGEIYPRSGNTYFNEKFKGKA TLTADKSSSTAYMELRSLTSEDSAVYFCARDRTGTGVDDYWGQGTTLTVSSASTKGPSVFPLAPSSKST SGGTAALGCLVKDYFPEPVTVSWNSGALTSGVHTFPAVLQSSGLYSLSSVVTVPSSSLGTQTYICNVNH KPSNTKVDKKVEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPPVLDSDGSFFLYS KLTVDKSRWQQGNVFSCSVMHEALHNHYTQKSLSLSPGK (SEQ ID NO: 33)<br><br>caggtgcagctgcagcagtccggcaccgagctggctaggccccggagcttccgtgaagctgagctgcaaggcctccggctataccttcaccagctacggcatca cctgggtgaagcagaggaccggccagggcctggagtggatcggcgagatctatcctaggagcggcaacacctacttcaacgagaagttcaagggcaaggcta ccctgaccgccgacaagtcctcctccaccgcctatatggagctgaggtccctgacctccgaggatagcgctgtgtacttctgcgctcgggacaggaccggcacc ggcgttgatgactactggggccagggcaccaccctgaccgtgagctccgcctccaccaagggccctccgtgttccctctggctccttcctccaagtccaccagc ggcggcaccgctgccctgggatgtctggtgaaggactacttccctgagcccgtgaccgtgtcttggaatagcggcgctctgacctccggcgtgcacaccttcccc gccgtgctgcagtccagcggcctgtatagcctgtccagcgtggtgaccgtgcccagcagctccctgggcacccagacctacatctgtaatgtgaatcacaagcc cagcaacaccaaggtggacaagaaggtggagcccaagtcctgtgataagacccacacctgtcccccctgccctgcccctgagctgctgggaggaccttccgtg ttcctgttcccccccaagcccaaggacaccctgatgatctcccggacccctgaggtgacctgtgtggtggtggatgtgtcccacgaggaccctgaggtgaagttc aattggtatgtggacggcgtggaggtgcacaatgccaagaccaagcccaggggaggagcagtacaacagcaccatcgggtggtgtccgtgctgaccgtgctgc accaggactggctgaacggcaaggagtacaagtgcaaggtgtccaacaaggccctgcctgctcccatcgagaagaccatcagcaaggccaagggccagccc agggagccccaggtgtataccctgcctcctagccgggacgagctgaccaagaaccaggtgagcctgacctgtctggtgaaaggcttctatcctagcgacatcgc cgtggagtgggagtccaatggccagcctgagaataattacaagaccaccctcccgtgctggacagcgacggctccttcttcctgtatagcaagctgaccgtgga taagagcaggtggcagcagggcaatgtgttctcctgttccgtgatgcacgaggctctgcacaatcactacacccagaagagcctgagcctgagccccggcaag (SEQ ID NO: 34) |
| Light chain of ch3G9B1<br><br>DIVLTQSPASLSMAIGEKVTIRCITNTDIDDDMSWYQQKPGEPPNLLISEGNTLRPGVPSRFSSSGYGRDF VFTIENMLSEDVADYYCLQSNNLPYTFGGGTKLEIKRTVAAPSVFIFPPSDEQLKSGTASVVCLLNNFYP REAKVQWKVDNALQSGNSQESVTEQDSKDSTYSLSSTLTLSKADYEKHKVYACEVTHQGLSSPVTKS FNRGEC (SEQ ID NO: 35)<br><br>gacatcgtgctgacccagagccctgcctccctgagcatggccatcggcgagaaggtgaccatccggtgtatcaccaacaccgacatcgatgacgacatgagct ggtaccagcagaagcctggcgagccccctaacctgctgatctccgagggcaacaccctgaggcccggcgtgccttccaggttcagctccagcggctatggcag ggacttcgtgttcaccatcgagaacatgctgagcgaggacgtggccgactattactgcctgcagtccaataacctgccttacaccttcggcggcggcaccaagct ggagatcaagcgtacggtggctgcccctagcgtgttcatcttcccccctagcgatgagcagctgaagtccggcaccgccagcgtggtgtgcctgctgaataactt ctaccctagggaggccaaggtgcagtggaaggtggataacgccctgcagagcggcaactcccaggagagcgtgaccgagcaggacagcaaggattccacct attccctgtccagcaccctgaccctgagcaaggccgattatgagaagcacaaggtgtacgcctgcgaggtgacccaccagggcctgtccagccctgtgaccaa gtccttcaacaggggcgagtgt (SEQ ID NO: 36) |
| Heavy chain of ch29H5D8 |

(continued)

| Description |
| --- |
| Sequence (SEQ ID NO:) |
| EVQLQQSGAELARPGASVKLSCKASGYTFTNYGISWVKQRTGQGLEWIGEIYPRSVNTYYSAKFKGK ATLTADKSSSTAYMELRSLTSEDSAVYFCARHYYGSSLDYWGQGTTLTVSSASTKGPSVFPLAPSSKSTS GGTAALGCLVKDYFPEPVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK (SEQ ID NO: 37) |
| gaggtgcagctgcagcagtccggcgctgagctggctcggcctggagctagcgtgaagctgagctgtaaggcttccggctacaccttcaccaactatggcatcag ctgggtgaagcagaggaccggccagggcctggagtggatcggcgagatctaccccaggtccgtgaatacctactattccgccaagttcaagggcaaggctacc ctgaccgctgacaagagcagctccaccgcctacatggagctgcggtccctgacctccgaggatagcgctgtgtacttctgcgcccggcactactatggcagcag cctggattactggggccagggcaccaccctgaccgtgagctccgcctccaccaagggcccctccgtgttccctctggctccttcctccaagtccaccagcggcg gcaccgctgccctgggatgtctggtgaaggactacttccctgagcccgtgaccgtgtcttggaatagcggcgctctgacctccggcgtgcacaccttccccgccg tgctgcagtccagcggcctgtatagcctgtccagcgtggtgaccgtgcccagcagctccctgggcacccagacctacatctgtaatgtgaatcacaagcccagca acaccaaggtggacaagaaggtggagcccaagtcctgtgataagacccacacctgtccccctgccctgcccctgagctgctgggaggaccttccgtgttcctg ttccccccaagcccaaggacaccctgatgatctcccggacccctgaggtgacctgtgtggtggtggatgtgtcccacgaggaccctgaggtgaagttcaattgg tatgtggacggcgtggaggtgcacaatgccaagaccaagcccagggaggagcagtacaacagcacctatcgggtggtgtccgtgctgaccgtgctgcaccag gactggctgaacggcaaggagtacaagtgcaaggtgtccaacaaggccctgcctgctccatcgagaagaccatcagcaaggccaagggccagcccaggga gccccaggtgtataccctgcctcctagccgggacgagctgaccaagaaccaggtgagcctgacctgtctggtgaaaggcttctatcctagcgacatcgccgtgg agtgggagtccaatggccagcctgagaataattacaagaccacccctcccgtgctggacagcgacggctccttcttcctgtatagcaagctgaccgtggataaga gcaggtggcagcagggcaatgtgttctcctgttccgtgatgcacgaggctctgcacaatcactacacccagaagagcctgagcctgagccccggcaag (SEQ ID NO: 38) |

| Light chain of ch29H5D8 |
| --- |
| DIVLTQSPASLSVATGEKVTIRCITSSDIEDDMNWYQQKPGEPPKLLISEGNSLRPGVPSRFSSSGYGTDF VFTIENTLSEDVADYYCLQSDNIFTFGSGTKLEIKRTVAAPSVFIFPPSDEQLKSGTASVVCLLNNFYPRE AKVQWKVDNALQSGNSQESVTEQDSKDSTYSLSSTLTLSKADYEKHKVYACEVTHQGLSSPVTKSFN RGEC (SEQ ID NO: 39) |
| gatatcgtgctgacccagtcccccgcctccctgtccgtggctaccggagagaaggtgaccatccggtgtatcacctcctccgatatcgaggacgacatgaactgg tatcagcagaagcccggcgagcccctaagctgctgatctccgagggcaatagcctgaggcccggcgtgcctagccggttctcctcctccggctacggcaccg acttcgtgttcaccatcgagaacaccctgagcgaggatgtggccgactactactgtctgcagagcgacaacatcttcaccttcggctccggcaccaagctggaga tcaagcgtacggtggctgcccctagcgtgttcatcttccccctagcgatgagcagctgaagtccggcaccgccagcgtggtgtgcctgctgaataacttctaccc tagggaggccaaggtgcagtggaaggtggataacgccctgcagagcggcaactcccaggagagcgtgaccgagcaggacagcaaggattccacctattccc tgtccagcaccctgaccctgagcaaggccgattatgagaagcacaaggtgtacgcctgcgaggtgacccaccagggcctgtccagccctgtgaccaagtccttc aacaggggcgagtgt (SEQ ID NO: 40) |

| Heavy chain of ch135A2B10 |
| --- |
| DVKLVESGGGLVTPEGSLKLSCAASGFAFSTYGMAWVRQTPEKRLEWVASIHKGGFSTYYPDNVKGR FTISRDNDKNTLYLQMSSLKSEDTAMYYCIRHDWDGAMDYWGQGTSVTVSSASTKGPSVFPLAPSSK STSGGTAALGCLVKDYFPEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO: 41) |

| Description |
| --- |
| Sequence (SEQ ID NO:) |

| |
| --- |
| gacgtgaagctggtggagagcggcggcggcctggtgacacctgagggaagcctgaagctgagctgcgccgcctccggcttcgccttctccacctacggcatg gcttggggtgcggcagacccctgagaagcggctggagtggggtggccagcatccacaagggcggcttcagcacctactaccccgacaacgtgaagggccggttc accatctcccgggataatgataagaacaccctgtatctgcagatgtccagcctgaagtccgaggacaccgccatgtactactgcatccggcacgactgggacgg cgctatggattattggggccagggcaccagcgtgaccgtgtccagcgcctccaccaagggcccctccgtgttccctctggcctccttcctccaagtccaccagcgg cggcaccgctgccctgggatgtctggtgaaggactacttccctgagcccgtgaccgtgtcttggaatagcggcgctctgacctccggcgtgcacaccttccccgc cgtgctgcagtccagcggcctgtatagcctgtccagcgtggtgaccgtgcccagcagctccctgggcacccagacctacatctgtaatgtgaatcacaagccca gcaacaccaaggtggacaagaaggtggagcccaagtcctgtgataagacccacacctgtcccccctgccctgcccctgagctgctgggaggaccttccgtgttc ctgttcccccccaagcccaaggacaccctgatgatctcccggacccctgaggtgacctgtgtggtggtggatgtgtcccacgaggaccctgaggtgaagttcaat tggtatgtggacggcgtggaggtgcacaatgccaagaccaagcccagggaggagcagtacaacagcacctatcgggtggtgtccgtgctgaccgtgctgcac caggactggctgaacggcaaggagtacaagtgcaaggtgtccaacaaggccctgcctgctcccatcgagaagaccatcagcaaggccaagggccagcccag ggagccccaggtgtatacctgcctcctagccgggacgagctgaccaagaaccaggtgagcctgacctgtctggtgaaaggcttctatcctagcgacatcgccg tggagtgggagtccaatggccagcctgagaataattacaagaccacccctcccgtgctggacagcgacggctccttcttcctgtatagcaagctgaccgtggata agagcaggtggcagcagggcaatgtgttctcctgttccgtgatgcacgaggctctgcacaatcactacacccagaagagcctgagcctgagccccggcaag (SEQ ID NO: 42) |

| |
| --- |
| Light chain of ch135A2B10 |
| DIVLTQSPSSMYASLGERVTITCKASQDINSYLNWFQQKPGKSPKTLIYRANRLVDGVPSRFSGSGSGQD YSLTISSLEYEDLGIYYCLQFDEFPYTFGGGTKLEIKRTVAAPSVFIFPPSDEQLKSGTASVVCLLNNFYP REAKVQWKVDNALQSGNSQESVTEQDSKDSTYSLSSTLTLSKADYEKHKVYACEVTHQGLSSPVTKS FNRGEC (SEQ ID NO: 43) |
| gatatcgtgctgacccagagcccttcctccatgtatgctagcctgggcgagagggtgaccatcacctgcaaggctagccaggatatcaatagctacctgaattggt tccagcagaagcctggcaagtcccccaagaccctgatctaccgggctaatcggctggtggacggcgtgcccagcaggttctccggctccggatccggccagg actacagcctgaccatctccagcctggagtacgaggacctgggcatctactactgcctgcagttcgatgagttcccctataccttcggcggcggcaccaagctgg agatcaagcgtacggtggctgcccctagcgtgttcatcttcccccctagcgatgagcagctgaagtccggcaccgccagcgtggtgtgcctgctgaataacttcta ccctagggaggccaaggtgcagtggaaggtggataacgccctgcagagcggcaactcccaggagagcgtgaccgagcaggacagcaaggattccacctatt ccctgtccagcaccctgaccctgagcaaggccgattatgagaagcacaaggtgtacgcctgcgaggtgacccaccagggcctgtccagccctgtgaccaagtc cttcaacaggggcgagtgt (SEQ ID NO: 44) |

| |
| --- |
| Heavy chain of ch137G11D10 |
| DVQLQESGPGLVKPSQSLSLTCTVTGYSITSDYAWNWIRQFPGNKLEWMGYITYSASTSYNPSLKSRISI TRDTSKNQFFLQLNSVTTEDTATYYCARGAGTYYFDYWGQGSTLTVSSASTKGPSVFPLAPSSKSTSG GTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK (SEQ ID NO: 45) |
| gatgtgcagctgcaggagtccggccctggcctggtgaagccctcccagagcctgtccctgacctgtaccgtgaccggctactccatcaccagcgattacgcttgg aattggatcaggcagttccccggcaacaagctggagtggatgggctacatcacctattccgctagcacctcctataatccctccctgaagagccggatctccatca cccgggacaccagcaagaaccagttcttcctgcagctgaacagcgtgaccaccgaggacaccgccacctattattgtgccaggggcgctggcacctattacttc gattattggggccagggcagcaccctgaccgtgagctccgcctccaccaagggcccctccgtgttccctctggcctccttcctccaagtccaccagcggcggcac cgctgccctgggatgtctggtgaaggactacttccctgagcccgtgaccgtgtcttggaatagcggcgctctgacctccggcgtgcacaccttccccgccgtgct gcagtccagcggcctgtatagcctgtccagcgtggtgaccgtgcccagcagctccctgggcacccagacctacatctgtaatgtgaatcacaagcccagcaaca ccaaggtggacaagaaggtggagcccaagtcctgtgataagacccacacctgtcccccctgccctgcccctgagctgctgggaggaccttccgtgttcctgttcc cccccaagcccaaggacaccctgatgatctcccggacccctgaggtgacctgtgtggtggtggatgtgtcccacgaggaccctgaggtgaagttcaattggtatg tggacggcgtggaggtgcacaatgccaagaccaagcccagggaggagcagtacaacagcacctatcgggtggtgtccgtgctgaccgtgctgcaccaggact ggctgaacggcaaggagtacaagtgcaaggtgtccaacaaggccctgcctgctcccatcgagaagaccatcagcaaggccaagggccagcccaggggagcc ccaggtgtatacctgcctcctagccgggacgagctgaccaagaaccaggtgagcctgacctgtctggtgaaaggcttctatcctagcgacatcgccgtggagt gggagtccaatggccagcctgagaataattacaagaccacccctcccgtgctggacagcgacggctccttcttcctgtatagcaagctgaccgtggataagagca ggtggcagcagggcaatgtgttctcctgttccgtgatgcacgaggctctgcacaatcactacacccagaagagcctgagcctgagccccggcaag (SEQ ID NO: 46) |
| Light chain of ch137G11D10 |

**EP 4 772 535 A1**

(continued)

| Description / Sequence (SEQ ID NO:) |
|---|
| DIVLTQSPASLSVATGEKVTIRCITSTDIDEDMNWYQQKPGEPPKLLISEDNSLRPGVPSRFSSSGYGTDF VLTIENTLSEDVADYYCLQTNNLPLTFGAGTKLEIKRTVAAPSVFIFPPSDEQLKSGTASVVCLLNNFYPR EAKVQWKVDNALQSGNSQESVTEQDSKDSTYSLSSTLTLSKADYEKHKVYACEVTHQGLSSPVTKSF NRGEC (SEQ ID NO: 47) |
| gacatcgtgctgacccagtccccgctagcctgtccgtggctaccggcgagaaggtgaccatccggtgtatcaccagcaccgatatcgacgaggatatgaactg gtaccagcagaagcctggcgagcctcctaagctgctgatctccgaggacaactccctgcggcctggcgtgccctccaggttcagctccagcggctacggcacc gacttcgtgctgaccatcgagaacaccctgtccgaggatgtggccgattattactgtctgcagaccaacaatctgcccctgaccttcggcgccggcaccaagctg gagatcaagcgtacggtggctgcccctagcgtgttcatcttcccccctagcgatgagcagctgaagtccggcaccgccagcgtggtgtgcctgctgaataacttct accctagggaggccaaggtgcagtggaaggtggataacgccctgcagagcggcaactccaggagagcgtgaccgagcaggacagcaaggattccacctat tccctgtccagcaccctgaccctgagcaaggccgattatgagaagcacaaggtgtacgcctgcgaggtgacccaccagggcctgtccagccctgtgaccaagt ccttcaacaggggcgagtgt (SEQ ID NO: 48) |
| HCDR2 of hz29H5D8-1.1, hz29H5D8-2.1, and hz29H5D8-3.1 EIYPRSVNTYYAQKFQG (SEQ ID NO: 49) |
| HCDR2 of m29H5D8 and chimeric and humanized antibodies thereof EIYPRSVNTYYX$_1$X$_2$KFX$_3$G (SEQ ID NO: 50, wherein X$_1$ is selected from the group consisting of S and A, X$_2$ is selected from the group consisting of A and Q, and X$_3$ is selected from the group consisting of K and Q) |
| VH of hz29H5D8-1.1, hz29H5D8-2.1, and hz29H5D8-3.1 QVQLVQSGAEVKKPGSSVKVSCKASGYTFTNYGISWVRQAPGQGLEWMGEIYPRSVNTYYAQKFQG RVTITADKSTSTAYMELSSLRSEDTAVYYCARHYYGSSLDYWGQGTLVTVSS (SEQ ID NO: 51) |
| VH of hz29H5D8-1.2, hz29H5D8-2.2, and hz29H5D8-3.2 QVQLVQSGAEVKKPGSSVKVSCKASGYTFTNYGISWVRQAPGQGLEWMGEIYPRSVNTYYSAKFKG RVTITADKSTSTAYMELSSLRSEDTAVYYCARHYYGSSLDYWGQGTLVTVSS (SEQ ID NO: 52) |
| VL of hz29H5D8-1.1 and hz29H5D8-1.2 ETTLTQSPAFMSATPGDKVNISCITSSDIEDDMNWYQQKPGEAAILIISEGNSLRPGIPPRFSSSGYGTDFT LTINNIESEDAAYYFCLQSDNIFTFGQGTKVEIK (SEQ ID NO: 53) |
| VL of hz29H5D8-2.1 and hz29H5D8-2.2 DIQMTQSPSSLSASVGDRVTITCITSSDIEDDMNWYQQKPGKAPKLLIYEGNSLRPGVPSRFSSSGYGTD FTFTISSLQPEDIATYYCLQSDNIFTFGQGTKVEIK (SEQ ID NO: 54) |
| VL of hz29H5D8-3.1 and hz29H5D8-3.2 DIQMTQSPSSLSASVGDRVTITCITSSDIEDDMNWYQQKPGKAPKLLISEGNSLRPGVPSRFSSSGYGTD FTFTISSLQPEDIATYYCLQSDNIFTFGQGTKVEIK (SEQ ID NO: 55) |
| VH of hz135A2B10-1.1 QVQLVESGGGLVQPGGSLRLSCAASGFAFSTYGMAWVRQAPGKGLEWVASIHKGGFSTYYPDNVKGR FTISRDNAKNSLYLQMNSLRAEDTAVYYCARHDWDGAMDYWGQGTLVTVSS (SEQ ID NO: 56) |
| VH of hz135A2B10-1.2 QVQLVESGGGLVQPGGSLRLSCAASGFAFSTYGMAWVRQAPGKGLEWVASIHKGGFSTYYPDNVKGR FTISRDNDKNSLYLQMNSLRAEDTAVYYCIRHDWDGAMDYWGQGTLVTVSS (SEQ ID NO: 57) |
| VH of hz135A2B10-1.3 QVQLVESGGGLVQPGGSLRLSCAASGFAFSTYGMAWVRQAPGKRLEWVASIHKGGFSTYYPDNVKGR FTISRDNDKNSLYLQMNSLRAEDTAVYYCIRHDWDGAMDYWGQGTLVTVSS (SEQ ID NO: 58) |
| VL of hz135A2B 10-1.1, hz135A2B10-1.2, and hz135A2B10-1.3 DIQMTQSPSSLSASVGDRVTITCKASQDINSYLNWYQQKPGKAPKTLIYRANRLVDGVPSRFSGSGSGQ DYTLTISSLQPEDFATYYCLQFDEFPYTFGQGTKVEIK (SEQ ID NO: 59) |
| VH of hz137G11D10-1.1, hz137G11D10-2.1, and hz137G11D10-3.1 |

44

| Description<br>Sequence (SEQ ID NO:) |
|---|
| QVQLQESGPGLVKPSQTLSLTCTVSGYSITSDYAWNWIRQHPGKGLEWIGYITYSASTSYNPSLKSRVTI SRDTSKNQFSLKLSSVTAADTAVYYCARGAGTYYFDYWGQGTLVTVSS (SEQ ID NO: 60) |
| VH of hz137G11D10-1.2, hz137G11D10-2.2, and hz137G11D10-3.2<br>QVQLQESGPGLVKPSQTLSLTCTVSGYSITSDYAWNWIRQHPGKGLEWIGYITYSASTSYNPSLKSRVTI SRDTSKNQFFLKLSSVTAADTAVYYCARGAGTYYFDYWGQGTLVTVSS (SEQ ID NO: 61) |
| VL of hz137G11D10-1.1 and hz137G11D10-1.2<br>ETTLTQSPAFMSATPGDKVNISCITSTDIDEDMNWYQQKPGEAAKLIISEDNSLRPGIPPRFSSSGYGTDF TLTINNIESEDAAYYFCLQTNNLPLTFGQGTKVEIK (SEQ ID NO: 62) |
| VL of hz137G11D10-2.1 and hz137G11D10-2.2<br>DIQMTQSPSSLSASVGDRVTITCITSTDIDEDMNWYQQKPGKAPKLLIYEDNSLRPGVPSRFSGSGYGT DFTLTISSLQPEDFATYYCLQTNNLPLTFGQGTKVEIK (SEQ ID NO: 63) |
| VL of hz137G11D10-3.1 and hz137G11D10-3.2<br>DIQMTQSPSSLSASVGDRVTITCITSTDIDEDMNWYQQKPGKAPKLLISEDNSLRPGVPSRFSSSGYGTD FTLTISSLQPEDFATYYCLQTNNLPLTFGQGTKVEIK (SEQ ID NO: 64) |
| Heavy chain of hz29H5D8-1.1, hz29H5D8-2.1, and hz29H5D8-3.1<br>QVQLVQSGAEVKKPGSSVKVSCKASGYTFTNYGISWVRQAPGQGLEWMGEIYPRSVNTYYAQKFQG RVTITADKSTSTAYMELSSLRSEDTAVYYCARHYYGSSLDYWGQGTLVTVSSASTKGPSVFPLAPSSKS TSGGTAALGCLVKDYFPEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO: 65)<br><br>caagtgcaactagtgcaaagtggtgcagaagtcaagaagcccggatcctccgtgaaagtgtcctgcaaggcctctggctacaccttcaccaactacggcatctcc tgggtgcggcaggctcctggccagggcctggagtggatgggcgagatctaccctagatctgtgaacacctactacgcccagaagttccagggaagagtgacca tcaccgccgacaagtccacaagcacagcctacatggaactgtcctctctgcggtccgaggataccgctgtgtactactgtgctagacactactatggctctagcct ggactactggggccaaggcaccctggtgaccgtgtctagcgcctccaccaagggcccttccgtgttcccactggctccctcttccaagtctacatccggaggaac cgccgctctgggatgcctggtgaaggattatttcccagagcccgtgaccgtgtcttggaactccggcgccctgacaagcggagtgcatacctttcctgctgtgctg cagagctctggcctgtattctctgtccagcgtggtgacagtgccatcttccagcctgggcacccagacatacatctgcaacgtgaatcacaagcctagcaatacca aggtggacaagaaggtggagccaaagtcttgtgataagacccatacatgcccccccttgtcctgctccagagctgctgggaggaccatccgtgttcctgtttccacc caagcccaaggacaccctgatgatctcccgcacaccagaggtgacctgcgtggtggtggacgtgagccacgaggatcccgaggtgaagtttaactggtacgtg gatggcgtggaggtgcataatgctaagaccaagccaagagaggagcagtataacagcacataccgcgtggtgtctgtgctgaccgtgctgcaccaggactggc tgaacggcaaggagtacaagtgcaaggtgtctaataaggccctgcccgctcctatcgagaagacaatctccaaggccaagggccagcctaggggagccacagg tgtataccctgcctccatctcgggacgagctgacaaagaaccaggtgtccctgacctgtctggtgaagggcttctacccccagcgatatcgctgtggagtgggagtc taatggccagcctgagaacaattataagaccacaccccctgtgctggacagcgatggctctttctttctgtactctaagctgacagtggataagtccaggtggcagc agggcaacgtgtttagctgctctgtgatgcatgaggctctgcacaatcattacacccagaagtccctgagcctgtctcccggcaag (SEQ ID NO: 66) |
| Heavy chain of hz29H5D8-1.2, hz29H5D8-2.2, and hz29H5D8-3.2<br>QVQLVQSGAEVKKPGSSVKVSCKASGYTFTNYGISWVRQAPGQGLEWMGEIYPRSVNTYYSAKFKG RVTITADKSTSTAYMELSSLRSEDTAVYYCARHYYGSSLDYWGQGTLVTVSSASTKGPSVFPLAPSSKS TSGGTAALGCLVKDYFPEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTPEVTCVVVDVSHEDPE<br><br>VKFNWYVDGVEVHNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO: 67) |

(continued)

| Description |
|---|
| Sequence (SEQ ID NO:) |

| caagtgcaactagtgcaaagtggtgcagaagtcaagaagcctggctcttctgtgaaagtgtcctgcaaggcctctggctacaccttcaccaactacggcatctcct gggtgcggcaggctcctggacagggcctggagtggatgggcgagatctaccccggtccgtgaacacctactactccgccaagttcaagggcagagtgacca tcaccgctgataagtccacatctacagcctacatggaactgtcctctctgagatccgaggacaccgccgtgtactactgtgctagacactactatggatccagcctg gactactggggccagggcaccctggtgaccgtgtctagcgcctccaccaagggcccttccgtgttcccactggctccctcttccaagtctacatccggaggaacc gccgctctgggatgcctggtgaaggattatttcccagagcccgtgaccgtgtcttggaactccggcgccctgacaagcggagtgcatacctttcctgctgtgctgc agagctctggcctgtattctctgtccagcgtggtgacagtgccatcttccagcctgggcacccagacatacatctgcaacgtgaatcacaagcctagcaataccaa ggtggacaagaaggtggagccaaagtcttgtgataagacccatacatgcccccccttgtcctgctccagagctgctgggaggaccatccgtgttcctgtttccaccc aagcccaaggacaccctgatgatctcccgcacaccagaggtgacctgcgtggtggtggacgtgagccacgaggatcccgaggtgaagtttaactggtacgtgg atggcgtggaggtgcataatgctaagaccaagccaagagaggagcagtataacagcacataccgcgtggtgtctgtgctgaccgtgctgcaccaggactggct gaacggcaaggagtacaagtgcaaggtgtctaataaggccctgccggctcctatcgagaagacaatctccaaggccaagggccagcctagggagccacaggt gtatacctgcctccatctcgggacgagctgacaaagaaccaggtgtccctgacctgtctggtgaagggcttctaccccagcgatatcgctgtggagtgggagtct aatggccagcctgagaacaattataagaccacacccctgtgctggacagcgatggctctttctttctgtactctaagctgacagtggataagtccaggtggcagca gggcaacgtgtttagctgctctgtgatgcatgaggctctgcacaatcattacacccagaagtccctgagcctgtctcccggcaag (SEQ ID NO: 68) |

| Light chain of hz29H5D8-1.1 and hz29H5D8-1.2 |
| ETTLTQSPAFMSATPGDKVNISCITSSDIEDDMNWYQQKPGEAAILIISEGNSLRPGIPPRFSSSGYGTDFT LTINNIESEDAAYYFCLQSDNIFTFGQGTKVEIKRTVAAPSVFIFPPSDEQLKSGTASVVCLLNNFYPREA KVQWKVDNALQSGNSQESVTEQDSKDSTYSLSSTLTLSKADYEKHKVYACEVTHQGLSSPVTKSFNR GEC (SEQ ID NO: 69) |
| gaaactactctaactcaaagtccagcatttatgtctgctacccctggcgacaaagtgaatatcagctgtatcacctcttctgacatcgaggacgacatgaactggtac cagcagaagcccggcgaggccgccatcctgatcatctccgaaggcaactccctgcggccaggaatccctcctagattctccagctctggctacggcaccgatttt acactgaccatcaacaacattgagtccgaggatgctgcctactacttctgcctgcagtccgacaacatcttcaccttcggccaaggcaccaaggtggaaatcaagc gtacggtggccgctcccagcgtgttcatctttccccctcctgacgagcagctgaagtctggcaccgcttccgtggtgtgcctgctgaacaatttctaccccagagag gccaaggtgcagtggaaggtggataacgctctgcagtccggcaatagccaggagtctgtgaccgagcaggactccaaggatagcacatattctctgtcttccacc ctgacactgtctaaggccgactacgagaagcacaaggtgtatgcttgcgaggtgacccatcagggcctgagctctcctgtgacaaagtcctttaatcgcggcgag tgt (SEQ ID NO: 70) |

| Light chain of hz29H5D8-2.1 and hz29H5D8-2.2 |
| DIQMTQSPSSLSASVGDRVTITCITSSDIEDDMNWYQQKPGKAPKLLIYEGNSLRPGVPSRFSSSGYGTD FTFTISSLQPEDIATYYCLQSDNIFTFGQGTKVEIKRTVAAPSVFIFPPSDEQLKSGTASVVCLLNNFYPRE AKVQWKVDNALQSGNSQESVTEQDSKDSTYSLSSTLTLSKADYEKHKVYACEVTHQGLSSPVTKSFN RGEC (SEQ ID NO: 71) |
| gatatccaaatgactcaaagtccaagtagtctgtccgcttctgtcggagatagagtgaccatcacctgtatcacctcctccgacatcgaggacgacatgaactggta tcagcagaagcccggcaaggcccctaagctgctgatctacgagggcaactccctgagacctggcgtgccatctcggttctccagctctggctacggcaccgact tcacctttacaatctcctctctgcagcctgaggatatcgccacctactactgcctgcaaagcgacaacatcttcaccttcggccagggcacaaaagtggaaatcaag cgtacggtggccgctcccagcgtgttcatctttccccctcctgacgagcagctgaagtctggcaccgcttccgtggtgtgcctgctgaacaatttctaccccagaga ggccaaggtgcagtggaaggtggataacgctctgcagtccggcaatagccaggagtctgtgaccgagcaggactccaaggatagcacatattctctgtcttcca ccctgacactgtctaaggccgactacgagaagcacaaggtgtatgcttgcgaggtgacccatcagggcctgagctctcctgtgacaaagtcctttaatcgcggcg agtgt (SEQ ID NO: 72) |

| Light chain of hz29H5D8-3.1 and hz29H5D8-3.2 |
| DIQMTQSPSSLSASVGDRVTITCITSSDIEDDMNWYQQKPGKAPKLLISEGNSLRPGVPSRFSSSGYGTD FTFTISSLQPEDIATYYCLQSDNIFTFGQGTKVEIKRTVAAPSVFIFPPSDEQLKSGTASVVCLLNNFYPRE AKVQWKVDNALQSGNSQESVTEQDSKDSTYSLSSTLTLSKADYEKHKVYACEVTHQGLSSPVTKSFN RGEC (SEQ ID NO: 73) |
| gatatccaaatgactcaaagtccaagtagtctgtctgcttctgtgggcgacagagtgaccatcacctgtatcacctccagcgatatcgaggacgacatgaactggta ccagcagaaacctggcaaggcccctaagctgctgatctccgagggcaactccctgagaccaggagtcccctctcggttctcctcctctggctacggcaccgactt cacatttacaatcagctctctgcagcctgaggatatcgccacctactactgcctgcaatccgacaacatcttcaccttcggccagggcaccaaggtggaaatcaag cgtacggtggccgctcccagcgtgttcatctttccccctcctgacgagcagctgaagtctggcaccgcttccgtggtgtgcctgctgaacaatttctaccccagaga ggccaaggtgcagtggaaggtggataacgctctgcagtccggcaatagccaggagtctgtgaccgagcaggactccaaggatagcacatattctctgtcttcca ccctgacactgtctaaggccgactacgagaagcacaaggtgtatgcttgcgaggtgacccatcagggcctgagctctcctgtgacaaagtcctttaatcgcggcg agtgt (SEQ ID NO: 74) |

(continued)

| Description Sequence (SEQ ID NO:) |
|---|

Heavy chain of hz135A2B10-1.1

QVQLVESGGGLVQPGGSLRLSCAASGFAFSTYGMAWVRQAPGKGLEWVASIHKGGFSTYYPDNVKGR
FTISRDNAKNSLYLQMNSLRAEDTAVYYCARHDWDGAMDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 75)

caagtgcaactagtggaaagtggtggtggtctcgtgcagcctggaggatctctgcggctgtcttgtgctgcttctggcttcgcctttctacctacggcatggcctgg
gtccgccaagctcctggcaagggcctggaatgggtggcctccatccacaagggcggcttctccacctattaccccgataatgtgaaaggcagattcaccatctcc
cgggacaacgccaagaactccctgtacctgcagatgaacagcctgagagccgaggacaccgccgtgtactactgcgccagacacgattgggacggcgctatg
gactactggggccagggcaccctggtgacagtgtccagcgcctccaccaagggcccttccgtgttcccactggctccctcttccaagtctacatccggaggaacc
gccgctctgggatgcctggtgaaggattatttcccagagcccgtgaccgtgtcttggaactccggcgccctgacaagcggagtgcatacctttcctgctgtgctgc
agagctctggcctgttctctgtccagcgtggtgacagtgccatcttccagcctgggcacccagacatacatctgcaacgtgaatcacaagcctagcaataccaa
ggtggacaagaaggtggagccaaagtcttgtgataagacccatacatgcccccttgtcctgctccagagctgctgggaggaccatccgtgttcctgtttccaccc
aagcccaaggacaccctgatgatctcccgcacaccagaggtgacctgcgtggtggtggacgtgagccacgaggatcccgaggtgaagtttaactggtacgtgg
atggcgtggaggtgcataatgctaagaccaagccaagagaggagcagtataacagcacataccgcgtggtgtctgtgctgaccgtgctgcaccaggactggct
gaacggcaaggagtacaagtgcaaggtgtctaataaggccctgcccgctcctatcgagaagacaatctccaaggccaagggccagcctagggagccacaggt
gtatacctgcctccatctcgggacgagctgacaaagaaccaggtgtccctgacctgtctggtgaagggcttctaccccagcgatatcgctgtggagtgggagtct
aatggccagcctgagaacaattataagaccacacccctgtgctggacagcgatggctctttctttctgtactctaagctgacagtggataagtccaggtggcagca
gggcaacgtgtttagctgctctgtgatgcatgaggctctgcacaatcattacacccagaagtccctgagcctgtctcccggcaag (SEQ ID NO: 76)

Heavy chain of hz135A2B10-1.2

QVQLVESGGGLVQPGGSLRLSCAASGFAFSTYGMAWVRQAPGKGLEWVASIHKGGFSTYYPDNVKGR
FTISRDNDKNSLYLQMNSLRAEDTAVYYCIRHDWDGAMDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 77)

caagtgcaactagtggaaagtggtggtggtctcgtgcagcctggaggaagcctgcggctgtcttgtgctgcttctggcttcgcctttctacatatggcatggcctgg
gtccgccaagctcctggcaagggcctggaatgggtggcctccatccacaagggcggcttctccacctactaccccgataatgtgaaaggcagattcaccatctcc
cgggacaacgacaagaactccctgtacctgcagatgaactctctgagagccgaggacaccgccgtgtactactgcatcagacacgattgggacggcgctatgg
actactggggccagggcaccctggtgaccgtgtccagcgcctccaccaagggcccttccgtgttcccactggctccctcttccaagtctacatccggaggaaccg
ccgctctgggatgcctggtgaaggattatttcccagagcccgtgaccgtgtcttggaactccggcgccctgacaagcggagtgcatacctttcctgctgtgctgca
gagctctggcctgttctctgtccagcgtggtgacagtgccatcttccagcctgggcacccagacatacatctgcaacgtgaatcacaagcctagcaataccaag
gtggacaagaaggtggagccaaagtcttgtgataagacccatacatgcccccttgtcctgctccagagctgctgggaggaccatccgtgttcctgtttccaccca
agcccaaggacaccctgatgatctcccgcacaccagaggtgacctgcgtggtggtggacgtgagccacgaggatcccgaggtgaagtttaactggtacgtgga
tggcgtggaggtgcataatgctaagaccaagccaagagaggagcagtataacagcacataccgcgtggtgtctgtgctgaccgtgctgcaccaggactggctg
aacggcaaggagtacaagtgcaaggtgtctaataaggccctgcccgctcctatcgagaagacaatctccaaggccaagggccagcctagggagccacaggtg
tatacctgcctccatctcgggacgagctgacaaagaaccaggtgtccctgacctgtctggtgaagggcttctaccccagcgatatcgctgtggagtgggagtcta
atggccagcctgagaacaattataagaccacacccctgtgctggacagcgatggctctttctttctgtactctaagctgacagtggataagtccaggtggcagca
gggcaacgtgtttagctgctctgtgatgcatgaggctctgcacaatcattacacccagaagtccctgagcctgtctcccggcaag (SEQ ID NO: 78)

Heavy chain of hz135A2B10-1.3

QVQLVESGGGLVQPGGSLRLSCAASGFAFSTYGMAWVRQAPGKRLEWVASIHKGGFSTYYPDNVKGR
FTISRDNDKNSLYLQMNSLRAEDTAVYYCIRHDWDGAMDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGK (SEQ ID NO: 79)

(continued)

| Description |
| --- |
| Sequence (SEQ ID NO:) |

caagtgcaactagtggaaagtggtggtggtctcgtgcagcctggcggctctctgagactgtcttgtgctgcttctggcttcgccttctccacatacggcatggcctg ggtcagacaagctcccggcaagcggctggaatgggtggcctccatccacaaaggaggatttttctacctactatcctgacaacgtgaagggcagattcaccatctc cagagataatgacaagaactccctgacctgcagatgaacagcctgcgggccgaggacaccgccgtgtactactgcatccggcacgattgggacggcgctatg gactactggggccagggcaccctggtgaccgtgtccagcgcctccaccaagggcccttccgtgttcccactggctccctcttccaagtctacatccggaggaacc gccgctctgggatgcctggtgaaggattatttcccagagcccgtgaccgtgtcttggaactccggcgccctgacaagcggagtgcataccttcctgctgtgctgc agagctctggcctgtattctctgtccagcgtggtgacagtgccatcttccagctgggcacccagacatacatctgcaacgtgaatcacaagcctagcaataccaa ggtggacaagaaggtggagccaaagtcttgtgataagacccatacatgccccccttgtcctgctccagagctgctgggaggaccatccgtgttcctgtttccaccc aagcccaaggacaccctgatgatctcccgcacaccagaggtgacctgcgtggtggtggacgtgagccacgaggatcccgaggtgaagtttaactggtacgtgg atggcgtggaggtgcataatgctaagaccaagccaagagaggagcagtataacagcacataccgcgtggtgtctgtgctgaccgtgctgcaccaggactggct gaacggcaaggagtacaagtgcaaggtgtctaataaggccctgcccgctcctatcgagaagacaatctccaaggccaagggccagcctagggagccacaggt gtataccctgcctccatctcgggacgagctgacaaagaaccaggtgtccctgacctgtctggtgaagggcttctaccccagcgatatcgctgtggagtgggagtct aatggccagcctgagaacaattataagaccacaccccctgtgctggacagcgatggctctttctttctgtactctaagctgacagtggataagtccaggtggcagca gggcaacgtgtttagctgctctgtgatgcatgaggctctgcacaatcattacacccagaagtccctgagcctgtctcccggcaag (SEQ ID NO: 80)

Light chain of hz135A2B10-1.1, hz135A2B10-1.2, and hz135A2B10-1.3

DIQMTQSPSSLSASVGDRVTITCKASQDINSYLNWYQQKPGKAPKTLIYRANRLVDGVPSRFSGSGSGQ DYTLTISSLQPEDFATYYCLQFDEFPYTFGQGTKVEIKRTVAAPSVFIFPPSDEQLKSGTASVVCLLNNFY PREAKVQWKVDNALQSGNSQESVTEQDSKDSTYSLSSTLTLSKADYEKHKVYACEVTHQGLSSPVTK SFNRGEC (SEQ ID NO: 81)

gatatccaaatgactcaaagtccaagtagtctgtccgcctccgtgggcgacagagtcacaatcacctgtaaggcctcccaggatatcaactcctacctgaactggt accagcagaagcctggcaaggctcctaagaccctgatctacagagccaaccggctggtggacggcgtgccatctcggttctccggctctggatctggccaagat tatacactgaccatctccagcctccagcctgaggacttcgccacctactactgcctgcagttcgacgagtttccctacaccttcggccagggcaccaaggtggaaa tcaagcgtacggtggccgctcccagcgtgttcatctttccccccttctgacgagcagctgaagtctggcaccgcttccgtggtgtgcctgctgaacaatttctacccca gagaggccaaggtgcagtggaaggtggataacgctctgcagtccggcaatagccaggagtctgtgaccgagcaggactccaaggatagcacatattctctgtct tccaccctgacactgtctaaggccgactacgagaagcacaaggtgtatgcttgcgaggtgacccatcagggcctgagctctcctgtgacaaagtcctttaatcgcg gcgagtgt (SEQ ID NO: 82)

Heavy chain of hz137G11D10-1.1, hz137G11D10-2.1, and hz137G11D10-3.1

QVQLQESGPGLVKPSQTLSLTCTVSGYSITSDYAWNWIRQHPGKGLEWIGYITYSASTSYNPSLKSRVTI SRDTSKNQFSLKLSSVTAADTAVYYCARGAGTYYFDYWGQGTLVTVSSASTKGPSVFPLAPSSKSTSG GTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK (SEQ ID NO: 83)

caagtgcaactacaagaaagtggtccaggtctggtgaagccttctcagaccctgtctctgacctgtaccgtgtctggctactccatcacctctgactacgcctggaa ctggatccggcaacacctggcaaaggactcgagtggatcggctacatcacatattctgcctctaccagctacaaccccagcctgaagtccagagtgaccatctc ccggggacacctccaagaaccagttctccctgaagctgtcctccgtgaccgctgctgataccgccgtgtactactgcgccagaggcgctggcacctactacttcga ctactggggccagggcacactggtcacagtgtccagcgcctccaccaagggcccttccgtgttcccactggctccctcttccaagtctacatccggaggaaccgc cgctctgggatgcctggtgaaggattatttcccagagcccgtgaccgtgtcttggaactccggcgccctgacaagcggagtgcataccttcctgctgtgctgcag agctctggcctgtattctctgtccagcgtggtgacagtgccatcttccagctgggcacccagacatacatctgcaacgtgaatcacaagcctagcaataccaagg tggacaagaaggtggagccaaagtcttgtgataagacccatacatgccccccttgtcctgctccagagctgctgggaggaccatccgtgttcctgtttccacccaa gcccaaggacaccctgatgatctcccgcacaccagaggtgacctgcgtggtggtggacgtgagccacgaggatcccgaggtgaagtttaactggtacgtggat ggcgtggaggtgcataatgctaagaccaagccaagagaggagcagtataacagcacataccgcgtggtgtctgtgctgaccgtgctgcaccaggactggctga acggcaaggagtacaagtgcaaggtgtctaataaggccctgcccgctcctatcgagaagacaatctccaaggccaagggccagcctagggagccacaggtgt ataccctgcctccatctcgggacgagctgacaaagaaccaggtgtccctgacctgtctggtgaagggcttctaccccagcgatatcgctgtggagtgggagtcta atggccagcctgagaacaattataagaccacaccccctgtgctggacagcgatggctctttctttctgtactctaagctgacagtggataagtccaggtggcagca gggcaacgtgtttagctgctctgtgatgcatgaggctctgcacaatcattacacccagaagtccctgagcctgtctcccggcaag (SEQ ID NO: 84)

Heavy chain of hz137G11D10-1.2, hz137G11D10-2.2, and hz137G11D10-3.2

(continued)

| Description |
|---|
| Sequence (SEQ ID NO:) |
| QVQLQESGPGLVKPSQTLSLTCTVSGYSITSDYAWNWIRQHPGKGLEWIGYITYSASTSYNPSLKSRVTI SRDTSKNQFFLKLSSVTAADTAVYYCARGAGTYYFDYWGQGTLVTVSSASTKGPSVFPLAPSSKSTSG GTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK (SEQ ID NO: 85) |
| caagtgcaactacaagaaagtggtccaggtctcgtgaagccttctcagaccctgtctctgacctgtacagtgtctggatactccatcacctctgactacgcctggaa ctggatccggcaacaccctggcaagggcctggagtggatcggctacatcacctattctgcctctacctcctacaaccccagcctgaaatccagagtgaccatctcc cgggacacctccaagaaccagttctttctgaagctgtcctccgtgacagctgctgataccgccgtgtactactgcgccagaggcgctggcacctactacttcgact actggggccagggcacactggtcaccgtgtccagcgcctccaccaagggcccttccgtgttcccactggctccctcttccaagtctacatccggaggaaccgcc gctctgggatgcctggtgaaggattatttcccagagcccgtgaccgtgtcttggaactccggcgccctgacaagcggagtgcatacctttcctgctgtgctgcaga gctctggcctgattctctgtcagcgtggtgacagtgccatcttccagcctgggcacccagacatacatctgcaacgtgaatcacaagcctagcaataccaaggt ggacaagaaggtggagccaaagtcttgtgataagacccatacatgccccccttgtcctgctccagagctgctgggaggaccatccgtgttcctgtttccacccaag cccaaggacaccctgatgatctcccgcacaccagaggtgacctgcgtggtggtggacgtgagccacgaggatcccgaggtgaagtttaactggtacgtggatg gcgtggaggtgcataatgctaagaccaagccaagagaggagcagtataacagcacataccgcgtggtgtctgtgctgaccgtgctgcaccaggactggctgaa cggcaaggagtacaagtgcaaggtgtctaataagccctgcccgctcctatcgagaagacaatctccaaggccaagggccagcctagggagccacaggtgtat accctgcctccatctcgggacgagctgacaaagaaccaggtgtccctgacctgtctggtgaagggcttctaccccagcgatatcgctgtggagtgggagtctaat ggccagcctgagaacaattataagaccacacccctgtgctggacagcgatggctctttctttctgtactctaagctgacagtggataagtccaggtggcagcagg gcaacgtgtttagctgctctgtgatgcatgaggctctgcacaatcattacacccagaagtccctgagcctgtctcccggcaag (SEQ ID NO: 86) |
| Light chain of hz137G11D10-1.1 and hz137G11D10-1.2 |
| ETTLTQSPAFMSATPGDKVNISCITSTDIDEDMNWYQQKPGEAAKLIISEDNSLRPGIPPRFSSSGYGTDF TLTINNIESEDAAYYFCLQTNNLPLTFGQGTKVEIKRTVAAPSVFIFPPSDEQLKSGTASVVCLLNNFYPR EAKVQWKVDNALQSGNSQESVTEQDSKDSTYSLSSTLTLSKADYEKHKVYACEVTHQGLSSPVTKSF NRGEC (SEQ ID NO: 87) |
| gaaactactctaactcaaagtccagcatttatgtccgctacccctggcgacaaggtgaacatttcttgtatcacctctacagatatcgacgaggacatgaactggtac cagcagaagcccggcgaggccgccaagctgatcatctccgaggacaactccctgagaccaggcatccctcctcggtttagcagctctggctacggcaccgact tcaccctgaccatcaacaatatcgagtccgaagatgctgcctactacttctgcctgcagaccaacaacctgcctctgacccttggccaaggaacaaaagtggaaat caagcgtacggtggccgctcccagcgtgttcatctttcccccttctgacgagcagctgaagtctggcaccgcttccgtggtgtgcctgctgaacaatttctacccca gagaggccaaggtgcagtggaaggtggataacgctctgcagtccggcaatagccaggagtctgtgaccgagcaggactccaaggatagcacatattctctgtct tccaccctgacactgtctaaggccgactacgagaagcacaaggtgtatgcttgcgaggtgacccatcagggcctgagctctcctgtgacaaagtcctttaatcgcg gcgagtgt (SEQ ID NO: 88) |
| Light chain of hz137G11D10-2.1 and hz137G11D10-2.2 |
| DIQMTQSPSSLSASVGDRVTITCITSTDIDEDMNWYQQKPGKAPKLLIYEDNSLRPGVPSRFSGSGYGT DFTLTISSLQPEDFATYYCLQTNNLPLTFGQGTKVEIKRTVAAPSVFIFPPSDEQLKSGTASVVCLLNNFY PREAKVQWKVDNALQSGNSQESVTEQDSKDSTYSLSSTLTLSKADYEKHKVYACEVTHQGLSSPVTK SFNRGEC (SEQ ID NO: 89) |
| gatatccaaatgactcaaagtccaagtagtctctctgcttctgtcggcgacagagtgaccatcacctgtatcacctccaccgacatcgacgaggacatgaattggta tcagcagaaaccaggcaaggcccctaagctgctgatctacgaggacaactccctgcggcctggcgtgccctccagattcagcggatctggctacggcacagatt tcaccctgacaatctccagcctgcaacctgaggattttgccacctactactgcctgcagaccaacaacctgcctctgaccttcggccagggcaccaaggtggaaat caagcgtacggtggccgctcccagcgtgttcatctttcccccttctgacgagcagctgaagtctggcaccgcttccgtggtgtgcctgctgaacaatttctacccca gagaggccaaggtgcagtggaaggtggataacgctctgcagtccggcaatagccaggagtctgtgaccgagcaggactccaaggatagcacatattctctgtct tccaccctgacactgtctaaggccgactacgagaagcacaaggtgtatgcttgcgaggtgacccatcagggcctgagctctcctgtgacaaagtcctttaatcgcg gcgagtgt (SEQ ID NO: 90) |
| Light chain of hz137G11D10-3.1 and hz137G11D10-3.2 |
| DIQMTQSPSSLSASVGDRVTITCITSTDIDEDMNWYQQKPGKAPKLLISEDNSLRPGVPSRFSSSGYGTD FTLTISSLQPEDFATYYCLQTNNLPLTFGQGTKVEIKRTVAAPSVFIFPPSDEQLKSGTASVVCLLNNFYP REAKVQWKVDNALQSGNSQESVTEQDSKDSTYSLSSTLTLSKADYEKHKVYACEVTHQGLSSPVTKS FNRGEC (SEQ ID NO: 91) |

(continued)

| Description |
|---|
| Sequence (SEQ ID NO:) |
| gatatccaaatgactcaaagtccaagtagtctctctgcctccgtgggcgacagagtgaccatcacctgtatcacctccaccgacatcgacgaggacatgaattggt accagcagaaacctggcaaggcccctaagctgctgatctccgaggacaactccctgagaccaggagtccccagccggttctccagctctggctacggcacaga tttcaccctgacaatctcttctctgcaacctgaggattttgctacctactactgcctgcagaccaacaacctgcctctgaccttcggccagggcaccaaggtggaaat caagcgtacggtggccgctcccagccgtgttcatctttcccccttctgacgagcagctgaagtctggcaccgcttccgtggtgtgcctgctgaacaattctaccccca gagaggccaaggtgcagtggaaggtggataacgctctgcagtccggcaatagccaggagtctgtgaccgagcaggactccaaggatagcacatattctctgtct tccaccctgacactgtctaaggccgactacgagaagcacaaggtgtatgcttgcgaggtgacccatcagggcctgagctctcctgtgacaaagtcctttaatcgcg gcgagtgt (SEQ ID NO: 92) |

| Human ROR1 ECD-hFc |
|---|
| QETELSVSAELVPTSSWNISSELNKDSYLTLDEPMNNITTSLGQTAELHCKVSGNPPPTIRWFKNDAPVV QEPRRLSFRSTIYGSRLRIRNLDTTDTGYFQCVATNGKEVVSSTGVLFVKFGPPPTASPGYSDEYEEDGF CQPYRGIACARFIGNRTVYMESLHMQGEIENQITAAFTMIGTSSHLSDKCSQFAIPSLCHYAFPYCDETS SVPKPRDLCRDECEILENVLCQTEYIFARSNPMILMRLKLPNCEDLPQPESPEAANCIRIGIPMADPINKN HKCYNSTGVDYRGTVSVTKSGRQCQPWNSQYPHTHTFTALRFPELNGGHSYCRNPGNQKEAPWCFTL DENFKSDLCDIPACDSKDSKEKNKMEIEGRMDPKSSDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSPGK (SEQ ID NO: 93) |

| Human/cynomolgus monkey ROR1 ECD-His |
|---|
| QETELSVSAELVPTSSWNISSELNKDSYLTLDEPMNNITTSLGQTAELHCKVSGNPPPTIRWFKNDAPVV QEPRRLSFRSTIYGSRLRIRNLDTTDTGYFQCVATNGKEVVSSTGVLFVKFGPPPTASPGYSDEYEEDGF CQPYRGIACARFIGNRTVYMESLHMQGEIENQITAAFTMIGTSSHLSDKCSQFAIPSLCHYAFPYCDETS SVPKPRDLCRDECEILENVLCQTEYIFARSNPMILMRLKLPNCEDLPQPESPEAANCIRIGIPMADPINKN HKCYNSTGVDYRGTVSVTKSGRQCQPWNSQYPHTHTFTALRFPELNGGHSYCRNPGNQKEAPWCFTL DENFKSDLCDIPACDSKDSKEKNKMEGGGSGGGSHHHHHHHHHH (SEQ ID NO: 94) |

| Human ROR1 |
|---|
| MHRPRRRGTRPPLLALLAALLLAARGAAAQETELSVSAELVPTSSWNISSELNKDSYLTLDEPMNNITT SLGQTAELHCKVSGNPPPTIRWFKNDAPVVQEPRRLSFRSTIYGSRLRIRNLDTTDTGYFQCVATNGKE VVSSTGVLFVKFGPPPTASPGYSDEYEEDGFCQPYRGIACARFIGNRTVYMESLHMQGEIENQITAAFT MIGTSSHLSDKCSQFAIPSLCHYAFPYCDETSSVPKPRDLCRDECEILENVLCQTEYIFARSNPMILMRLK LPNCEDLPQPESPEAANCIRIGIPMADPINKNHKCYNSTGVDYRGTVSVTKSGRQCQPWNSQYPHTHT FTALRFPELNGGHSYCRNPGNQKEAPWCFTLDENFKSDLCDIPACDSKDSKEKNKMEILYILVPSVAIPL AIALLFFFICVCRNNQKSSSAPVQRQPKHVRGQNVEMSMLNAYKPKSKAKELPLSAVRFMEELGECAF GKIYKGHLYLPGMDHAQLVAIKTLKDYNNPQQWTEFQQEASLMAELHHPNIVCLLGAVTQEQPVCML FEYINQGDLHEFLIMRSPHSDVGCSSDEDGTVKSSLDHGDFLHIAIQIAAGMEYLSSHFFVHKDLAARN ILIGEQLHVKISDLGLSREIYSADYYRVQSKSLLPIRWMPPEAIMYGKFSSDSDIWSFGVVLWEIFSFGLQ PYYGFSNQEVIEMVRKRQLLPCSEDCPPRMYSLMTECWNEIPSRRPRFKDIHVRLRSWEGLSSHTSSTT PSGGNATTQTTSLSASPVSNLSNPRYPNYMFPSQGITPQGQIAGFIGPPIPQNQRFIPINGYPIPPGYAAFPA AHYQPTGPPRVIQHCPPPKSRSPSSASGSTSTGHVTSLPSSGSNQEANIPLLPHMSIPNHPGGMGITVFGN KSQKPYKIDSKQASLLGDANIHGHTESMISAEL (SEQ ID NO: 95) |

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

**Claims**

1. An anti-ROR1 antibody or an antigen-binding fragment thereof, comprising: an HCDR1 comprising the amino acid sequence set forth in SEQ ID NO: 25, 1, 9, or 17, an HCDR2 comprising the amino acid sequence set forth in SEQ ID NO: 26, 2, 10, 18, 49, or 50, an HCDR3 comprising the amino acid sequence set forth in SEQ ID NO: 27, 3, 11, or 19, an LCDR1 comprising the amino acid sequence set forth in SEQ ID NO: 28, 4, 12, or 20, an LCDR2 comprising the amino acid sequence set forth in SEQ ID NO: 29, 5, 13, or 21, and an LCDR3 comprising the amino acid sequence set forth in SEQ ID NO: 30, 6, 14, or 22.

2. The anti-ROR1 antibody or the antigen-binding fragment thereof according to claim 1, wherein the anti-ROR1 antibody or the antigen-binding fragment thereof comprises:

   (1) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30;
   (2) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
   (3) an HCDR1 comprising the amino acid sequence set forth in SEQ ID NO: 9, an HCDR2 comprising the amino acid sequence set forth in SEQ ID NO: 50, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;
   (4) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;
   (5) an HCDR1 comprising the amino acid sequence set forth in SEQ ID NO: 9, an HCDR2 comprising the amino acid sequence set forth in SEQ ID NO: 49, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14; or
   (6) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

3. The anti-ROR1 antibody or the antigen-binding fragment thereof according to claim 1 or 2, wherein the anti-ROR1 antibody or the antigen-binding fragment thereof comprises a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, 7, 15, 23, 31, 51, 52, 56, 57, 58, or 61.

4. The anti-ROR1 antibody or the antigen-binding fragment thereof according to any one of claims 1-3, wherein the anti-ROR1 antibody or the antigen-binding fragment thereof comprises a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, 8, 16, 24, 32, 53, 54, 55, 59, 62, or 64.

5. The anti-ROR1 antibody or the antigen-binding fragment thereof according to any one of claims 1-4, wherein the anti-ROR1 antibody or the antigen-binding fragment thereof comprises:

(1) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63;

(2) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;

(3) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;

(4) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24;

(5) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32;

(6) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53;

(7) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54;

(8) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 51, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55;

(9) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 53;

(10) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 54;

(11) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 52, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 55;

(12) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 56, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(13) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 57, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(14) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 58, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 59;

(15) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62;

(16) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 60, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64;

(17) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62;

(18) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63; or

(19) a heavy chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 61, and a light chain variable region having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64.

6. The anti-ROR1 antibody or the antigen-binding fragment thereof according to any one of claims 1-5, wherein the antigen-binding fragment is a Fab fragment, a F(ab')2 fragment, an Fd fragment, an Fv fragment, an isolated CDR region, or an scFv;

optionally, the anti-ROR1 antibody or the antigen-binding fragment thereof is murine, chimeric, or humanized; optionally, the anti-ROR1 antibody is of the IgG1, IgG2, IgG3, or IgG4 isotype.

7. The anti-ROR1 antibody or the antigen-binding fragment thereof according to any one of claims 1-6, wherein the anti-ROR1 antibody comprises a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, 33, 37, 41, 45, 65, 67, 75, 77, 79, or 85.

8. The anti-ROR1 antibody or the antigen-binding fragment thereof according to any one of claims 1-7, wherein the anti-ROR1 antibody comprises a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89, 35, 39, 43, 47, 69, 71, 73, 81, 87, or 91.

9. The anti-ROR1 antibody or the antigen-binding fragment thereof according to any one of claims 1-8, wherein the anti-ROR1 antibody comprises:

(1) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89;

(2) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 33, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 35;

(3) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 37, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39;

(4) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 41, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 43;

(5) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 45, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 47;

(6) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

(7) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71;

(8) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73;

(9) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

(10) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 71;

(11) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 73;

(12) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 75, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(13) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 77, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(14) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 79, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 81;

(15) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83,

and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;
(16) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 83, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91;
(17) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 87;
(18) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 89; or
(19) a heavy chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 85, and a light chain having an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 91.

10. The anti-ROR1 antibody or the antigen-binding fragment thereof according to any one of claims 1-9, wherein the anti-ROR1 antibody or the antigen-binding fragment thereof has one or several of the following properties and characteristics:

(1) binding to human ROR1; preferably, binding to human ROR1 with $K_D$ of 5E-08 M or less, 1E-09 M or less, or 5E-10 M or less, as measured by surface plasmon resonance;
(2) binding to monkey ROR1; preferably, binding to human ROR1 with $K_D$ of 5E-08 M or less, 1E-09 M or less, or 5E-10 M or less, as measured by surface plasmon resonance;
(3) inhibiting the Wnt5a-stimulated non-classical Wnt signaling pathway, wherein preferably, the anti-ROR1 antibody or the antigen-binding fragment thereof has an inhibition rate of 10% or higher, 15% or higher, or 20% or higher against the Wnt5a-stimulated non-classical Wnt signaling pathway at a concentration of 20 nM;
(4) exerting an internalization effect in cells expressing ROR1;
(5) exerting an ADCC effect on cells expressing ROR1;
(6) inducing the regression of tumor growth *in vivo;* and
(7) delaying tumor growth *in vivo.*

11. An isolated nucleic acid, comprising a nucleotide sequence encoding the anti-ROR1 antibody or the antigen-binding fragment thereof according to any one of claims 1-10.

12. A method for preparing the anti-ROR1 antibody or the antigen-binding fragment thereof according to any one of claims 1-10, comprising culturing a host cell comprising the nucleic acid according to claim 11 to express the anti-ROR1 antibody or the antigen-binding fragment thereof, and isolating and purifying the anti-ROR1 antibody or the antigen-binding fragment thereof in the system.

13. An antibody-drug conjugate, comprising the anti-ROR1 antibody or the antigen-binding fragment thereof according to any one of claims 1-10.

14. A pharmaceutical composition, comprising the anti-ROR1 antibody or the antigen-binding fragment thereof according to any one of claims 1-10 or the antibody-drug conjugate according to claim 13, and one or more pharmaceutically acceptable excipients.

15. A method for treating a disease expressing ROR1, comprising administering to a subject in need a therapeutically effective amount of the anti-ROR1 antibody or the antigen-binding fragment thereof according to any one of claims 1-10, the antibody-drug conjugate according to claim 13, or the pharmaceutical composition according to claim 14, wherein

preferably, the disease is a tumor;
preferably, the tumor is leukemia, lymphoma, multiple myeloma, breast cancer, ovarian cancer, ovarian teratoma, colon cancer, lung cancer, skin cancer, pancreatic cancer, testicular cancer, bladder cancer, uterine cancer, prostate cancer, or adrenal cancer.

FIG. 1A

FIG. 1B

FIG. 1C

FIG. 2A

FIG. 2B

**Jeko-1**

× hz29H5D8-2.1
△ hz29H5D8-3.1
▽ hz29H5D8-3.2
□ UC-961

FIG. 2C

**SW620**

× hz29H5D8-2.1
△ hz29H5D8-3.1
▽ hz29H5D8-3.2
□ UC-961

FIG. 2D

PA-1

FIG. 2E

Jeko-1

FIG. 2F

**SW620**

Legend:
- ▼ hz135A2B10-1.1
- ▽ hz135A2B10-1.2
- △ hz135A2B10-1.3
- □ UC-961

FIG. 2G

**PA-1**

Legend:
- ● hz137G11D10-1.1
- ▼ hz137G11D10-1.2
- ◆ hz137G11D10-2.1
- ○ hz137G11D10-2.2
- ✕ hz137G11D10-3.1
- ▽ hz137G11D10-3.2
- □ UC-961

FIG. 2H

Jeko-1

FIG. 2I

SW620

FIG. 3A

**NCI-H1993-ROR1**

FIG. 3B

**MDA-MB-468-ROR1**

FIG. 3C

NCI-H1975-ROR1

FIG. 4

FIG. 5

# EP 4 772 535 A1

## INTERNATIONAL SEARCH REPORT

International application No.

**PCT/CN2024/114289**

### A. CLASSIFICATION OF SUBJECT MATTER

C07K16/28(2006.01)i; C12N15/13(2006.01)i; A61K39/395(2006.01)i; A61P35/00(2006.01)i; A61P35/02(2006.01)i

According to International Patent Classification (IPC) or to both national classification and IPC

### B. FIELDS SEARCHED

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

ATTXT, BETXT, CHTXT, CNTXT, DETXT, DWPI, EATXT, ENTXT, ENTXTC, EPTXT, ESTXT, FRTXT, GBTXT, JPTXT, KRTXT, LEXTXT, OETXT, RUTXT, SGTXT, TWTXT, USTXT, VEN, WOTXT, WPABSC, CNKI, 万方, WANFANG, 中国专利生物序列检索系统, China Patent Biological Sequence Search System, Genbank, ISI web of Science: 受体酪氨酸激酶样孤儿受体1, 抗体, 肿瘤, 白血病, 人源化, 申请人/发明人, Applicants/Inventors, antibody, ror1, ROR1, tyrosine kinase like orphan receptor, tumor, humanized, leukemia, cancer, SEQ ID NOs: 25-30

### C. DOCUMENTS CONSIDERED TO BE RELEVANT

| Category* | Citation of document, with indication, where appropriate, of the relevant passages | Relevant to claim No. |
|---|---|---|
| A | CN 114085288 A (EPIMAB BIOTHERAPEUTICS (SUZHOU) LIMITED et al.) 25 February 2022 (2022-02-25) <br> claims 1-11 and 23-25 | 1-15 (in part) |
| A | CN 110891973 A (UCL BUSINESS PLC) 17 March 2020 (2020-03-17) <br> claims 1-46 | 1-15 (in part) |
| A | CN 115819592 A (SHANGHAI PHARMACEUTICAL GROUP BIOLOGICAL THERAPY TECHNOLOGY CO., LTD. et al.) 21 March 2023 (2023-03-21) <br> claims 1-20 | 1-15 (in part) |
| A | KR 20210028556 A (LEGOCHEM BIOSCIENCES, INC. et al.) 12 March 2021 (2021-03-12) <br> claims 1-9 | 1-15 (in part) |
| A | US 2017233472 A1 (MACROGENICS, INC.) 17 August 2017 (2017-08-17) <br> claims 1-26 | 1-15 (in part) |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 November 2024** | **15 November 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/114289** |

### C. DOCUMENTS CONSIDERED TO BE RELEVANT

| Category* | Citation of document, with indication, where appropriate, of the relevant passages | Relevant to claim No. |
| --- | --- | --- |
| A | WO 2023020423 A1 (SHANDONG BOAN BIOTECHNOLOGY CO., LTD. et al.) 23 February 2023 (2023-02-23) claims 1-3 and 11-15 | 1-15 (in part) |
| A | WO 2022029660 A1 (JUNO THERAPEUTICS, INC.) 10 February 2022 (2022-02-10) claims 1-135 | 1-15 (in part) |

Form PCT/ISA/210 (second sheet) (July 2022)

**INTERNATIONAL SEARCH REPORT**

International application No.

**PCT/CN2024/114289**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/114289** |

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **15 (in part)**
because they relate to subject matter not required to be searched by this Authority, namely:

Claim 15 (in part) sets forth a method for treating a disease expressing ROR1, which falls within methods for treatment of diseases as defined in PCT Rule 39.1(iv). In the present report, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

Invention I: claims 1-15 (in part) relate to an ROR1 antibody or an antigen-binding fragment thereof, sequences of CDR1-3 of a heavy chain variable region thereof being SEQ ID NOs: 25-27, and sequences of CDR1-3 of a light chain variable region thereof being SEQ ID NOs: 28-30; and an isolated nucleic acid thereof, an antibody-drug conjugate, a pharmaceutical composition, and a preparation method for the antibody and the use thereof.

Invention II: claims 1-15 (in part) relate to an ROR1 antibody or an antigen-binding fragment thereof, sequences of CDR1-3 of a heavy chain variable region thereof being SEQ ID NOs: 1-3, and sequences of CDR1-3 of a light chain variable region thereof being SEQ ID NOs: 4-6; and an isolated nucleic acid thereof, an antibody-drug conjugate, a pharmaceutical composition, and a preparation method for the antibody and the use thereof.

Invention III: claims 1-15 (in part) relate to an ROR1 antibody or an antigen-binding fragment thereof, sequences of CDR1-3 of a heavy chain variable region thereof being SEQ ID NOs: 9, 50 and 11, and sequences of CDR1-3 of a light chain variable region thereof being SEQ ID NOs: 12-14; and an isolated nucleic acid thereof, an antibody-drug conjugate, a pharmaceutical composition, and a preparation method for the antibody and the use thereof.

Invention IV: claims 1-15 (in part) relate to an ROR1 antibody or an antigen-binding fragment thereof, sequences of CDR1-3 of a heavy chain variable region thereof being SEQ ID NOs: 9-11, and sequences of CDR1-3 of a light chain variable region thereof being SEQ ID NOs: 12-14; and an isolated nucleic acid thereof, an antibody-drug conjugate, a pharmaceutical composition, and a preparation method for the antibody and the use thereof.

Invention V: claims 1-15 (in part) relate to an ROR1 antibody or an antigen-binding fragment thereof, sequences of CDR1-3 of a heavy chain variable region thereof being SEQ ID NOs: 9, 49 and 11, and sequences of CDR1-3 of a light chain variable region thereof being SEQ ID NOs: 12-14; and an isolated nucleic acid thereof, an antibody-drug conjugate, a pharmaceutical composition, and a preparation method for the antibody and the use thereof.

Invention VI: claims 1-15 (in part) relate to an ROR1 antibody or an antigen-binding fragment thereof, sequences of CDR1-3 of a heavy chain variable region thereof being SEQ ID NOs: 17-19, and sequences of CDR1-3 of a light chain variable region thereof being SEQ ID NOs: 20-22; and an isolated nucleic acid thereof, an antibody-drug conjugate, a pharmaceutical composition, and a preparation method for the antibody and the use thereof.

The common feature comprised in inventions I to VI is an ROR1 antibody. However, the ROR1 antibody is known in the prior art. Therefore, inventions I to VI described above do not have a same or corresponding special technical feature, do not comply with PCT Rule 13.2, and lack unity of invention (PCT Rule 13.1).

Form PCT/ISA/210 (continuation of first sheet) (July 2022)

## INTERNATIONAL SEARCH REPORT

International application No.

**PCT/CN2024/114289**

| Box No. III | Observations where unity of invention is lacking (Continuation of item 3 of first sheet) |

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-15 (in part), which relate to an ROR1 antibody or an antigen-binding fragment thereof, sequences of CDR1-3 of a heavy chain variable region thereof being SEQ ID NOs: 25-27, and sequences of CDR1-3 of a light chain variable region thereof being SEQ ID NOs: 28-30**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

| International application No. |
| --- |
| **PCT/CN2024/114289** |

| Patent document cited in search report | | | Publication date (day/month/year) | Patent family member(s) | | | Publication date (day/month/year) |
| --- | --- | --- | --- | --- | --- | --- | --- |
| CN | 114085288 | A | 25 February 2022 | KR | 20230053602 | A | 21 April 2023 |
| | | | | MX | 2023002181 | A | 02 March 2023 |
| | | | | WO | 2022042488 | A1 | 03 March 2022 |
| | | | | CA | 3190117 | A1 | 03 March 2022 |
| | | | | US | 2024010753 | A1 | 11 January 2024 |
| | | | | TW | 202227491 | A | 16 July 2022 |
| | | | | IL | 300760 | A | 01 April 2023 |
| | | | | JP | 2023538945 | A | 12 September 2023 |
| | | | | AU | 2021334677 | A1 | 02 March 2023 |
| | | | | BR | 112023002895 | A2 | 21 March 2023 |
| | | | | EP | 4200336 | A1 | 28 June 2023 |
| | | | | EP | 4200336 | A4 | 25 September 2024 |
| CN | 110891973 | A | 17 March 2020 | GB | 201710835 | D0 | 16 August 2017 |
| | | | | JP | 2020530272 | A | 22 October 2020 |
| | | | | JP | 7242573 | B2 | 20 March 2023 |
| | | | | AU | 2018297630 | A1 | 16 January 2020 |
| | | | | WO | 2019008377 | A1 | 10 January 2019 |
| | | | | US | 2020354448 | A1 | 12 November 2020 |
| | | | | US | 11466083 | B2 | 11 October 2022 |
| | | | | EP | 3649152 | A1 | 13 May 2020 |
| | | | | US | 2023220073 | A1 | 13 July 2023 |
| | | | | MX | 2019015348 | A | 20 July 2020 |
| | | | | CA | 3068196 | A1 | 10 January 2019 |
| CN | 115819592 | A | 21 March 2023 | None | | | |
| KR | 20210028556 | A | 12 March 2021 | None | | | |
| US | 2017233472 | A1 | 17 August 2017 | WO | 2017142928 | A1 | 24 August 2017 |
| | | | | AR | 108034 | A1 | 11 July 2018 |
| | | | | TW | 201730212 | A | 01 September 2017 |
| | | | | UY | 37127 | A | 31 August 2017 |
| WO | 2023020423 | A1 | 23 February 2023 | WO | 2023206985 | A1 | 02 November 2023 |
| | | | | US | 2024342283 | A1 | 17 October 2024 |
| | | | | CN | 116964205 | A | 27 October 2023 |
| WO | 2022029660 | A1 | 10 February 2022 | KR | 20230095918 | A | 29 June 2023 |
| | | | | US | 2023324408 | A1 | 12 October 2023 |
| | | | | EP | 4192868 | A1 | 14 June 2023 |
| | | | | JP | 2023536326 | A | 24 August 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WARD et al.** *Nature*, 1989, vol. 341, 544-546
- **ELVIN A. KABAT et al.** Sequences of Proteins of Immunological Interest. National Institutes of Health, 1991
- **CYRUS CHOTHIA et al.** Canonical Structures for the Hypervariable Regions of Immunoglobulins. *J. Mol. Biol.*, 1987, vol. 196, 901-917